# EUROPEAN PATENT APPLICATION

(11) **EP 4 439 052 A1**
(43) Date of publication of application: **02.10.2024**
(21) Application number: 23193815.0
(22) Date of filing: 29.08.2023
(51) Int. Cl.: G01N 21/47, G01N 21/27, G01N 21/85, G01N 33/24, G01N 21/31, G01N 21/84

(54) **MATERIAL IMAGING SYSTEM AND METHOD**

(30) Priority: 31.03.2023 US 202363456438 P
(71) Applicant: Motion Metrics International Corp., Vancouver, British Columbia V6T 1Z3 (CA)
(72) Inventor: ALHUMSI, Obada, Vancouver (CA); MCKINLEY, Timothy A., Albany (US)
(74) Representative: Argyma

(57) **Abstract**

An imaging system 100 for earthen material 102 includes a support structure 104 adjacent an image location 106 for a pathway of earthen material exposed to varying and uncontrolled illumination 140 and optionally also to artificial illumination from an optional artificial illumination source 154 of the system, a spectral imager 108, and a reference device 149 each mounted to the support structure, wherein the spectral imager directed at the image location and arranged to measure an intensity of illumination reflected from earthen material at the image location.

## Description

### FIELD OF THE INVENTION

The specification relates generally to imaging, and, more specifically, to spectral imaging in varying and uncontrolled illumination.

### BACKGROUND

Spectral imaging is used to acquire spectral information or signatures about a material. Spectral imaging involves determining a reflectance of a material for a plurality of wavelength bands from an image of the material or a portion (e.g., a pixel) of an image of the material (e.g., a spectral response curve or graph). Each wavelength band represents a narrow wavelength range. The bands may be consecutive or spaced from one another. Consecutive bands may be used to gather a large amount of data. For some applications, a lesser amount of data is sufficient, and non-consecutive bands may be used. For example, reflectance information for only select bands of interest may be gathered where it is known that a material of interest exhibits characteristic reflectance features (e.g., absorption or emission lines) for those select bands of interest. The select bands of interest may be non-consecutive bands. The reflectance information for the select bands of interest may be sufficient to determine whether the material of interest is present.

The reflectance of a material is a function of the intensity of light received by the material. Reflectance is typically measured as a value between zero and one, where zero represents no reflectance (i.e., the material is completely absorbing the light) and one represents complete reflectance (i.e., the material is completely reflecting the light).

Determining the reflectance requires comparing the acquired intensity data for a wavelength or band of interest to the intensity of light received by the material for the wavelength or band of interest. In some applications, the material is exposed to controlled light with known intensity characteristics and a known position relative to the material, allowing for the intensity of light received by the material to be calculated.

However, in some applications, the material is exposed to uncontrolled illumination. The uncontrolled illumination may have an unknown and variable intensity at the wavelength or band of interest. For example, the material may be exposed to solar illumination which varies due to, e.g., solar emission variations, atmospheric variations (e.g., clouds), and environmental factors such as dust stirred up near the imaged material.

### SUMMARY

The following summary is intended to introduce the reader to various aspects of the applicant's teaching, but not to define any invention.

In one example, an imaging system for earthen material includes: a support structure adjacent an image location exposed to varying and uncontrolled illumination; a spectral imager mounted to the support structure, the spectral imager directed in a first direction at the image location and operable to measure an intensity of illumination reflected from the earthen material at the image location for each of a plurality of wavelength bands of interest; and at least one reference device directed in a second direction different from the first direction, the at least one reference device operable to sample the varying and uncontrolled illumination to which the image location is exposed for one or more reference wavelength bands.

In another example, an imaging system for earthen material includes: a support structure adjacent an image location exposed to varying and uncontrolled illumination; a spectral imager mounted to the support structure, the spectral imager directed in a first direction at the image location and operable to measure an intensity of illumination reflected from the earthen material at the image location for each of a plurality of wavelength bands of interest; at least one reference device directed in a second direction different from the first direction, the at least one reference device operable to sample the varying and uncontrolled illumination to which the image location is exposed for one or more reference wavelength bands; and a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to: direct the spectral imager to capture spectral imager data of an earthen material at the image location, the spectral imager data including the intensity of illumination reflected from the earthen material for each of the plurality of bands of interest; receive the spectral imager data from the spectral imager; direct the capture of illumination data from a sample of the varying and uncontrolled illumination sampled by the at least one reference device, the illumination data including the intensity of the varying and uncontrolled illumination for the one or more reference wavelength bands; receive the illumination data; apply the illumination data to determine an intensity of incident light at the image location for each of the plurality of bands of interest; and apply the spectral imager data and the intensity of incident light to determine a reflectance of the earthen material for each of the plurality of bands of interest.

In a further example, an imaging system for earthen material includes: a support structure adjacent an image location exposed to varying and uncontrolled illumination; a spectral imager mounted to the support structure, the spectral imager directed at and focused on the image location and operable to measure an intensity of illumination reflected from the image location for each of a plurality of bands of interest; and a reference object in a field of view of the spectral imager beyond the image location.

In yet a further example, an imaging system for earthen material includes: a support structure adjacent an image location exposed to varying and uncontrolled illumination; and a spectral imager mounted to the support structure, and wherein the spectral imager is operable to move between a first position and a second position , the first position directed in a first direction at and focused on the image location and in which the spectral imager is operable to measure an intensity of illumination reflected from the image location for each of a plurality of bands of interest, and the second position directed in a second direction different from the first direction and in which the spectral imager is operable to measure an intensity of the varying and uncontrolled illumination to which the image location is exposed for one or more reference wavelength bands.

In yet a further example, there is provided an imaging system for earthen material includes: a support structure adjacent an image location exposed to varying and uncontrolled illumination; a spectral imager mounted to the support structure, the spectral imager directed at the image location and operable to measure an intensity of illumination reflected from the image location for each of a plurality of bands of interest; a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to: direct the spectral imager to capture spectral imager data of an earthen material at the image location at a first time, the spectral imager data including the intensity of illumination reflected from the earthen material for each of the plurality of bands of interest; receive the spectral imager data from the spectral imager; direct the spectral imager to capture spectral imager data of a reference at a second time, the second time being different from the first time and the spectral imager data of the reference including the ratio of illumination reflected from the reference object for one or more reference wavelength bands; receive the spectral imager data of the reference from the spectral imager; apply the spectral imager data of the reference to determine an intensity of the varying and uncontrolled illumination at the second time for the one or more reference wavelength bands; apply the intensity of the varying and uncontrolled illumination at the second time to infer an intensity of incident light at the earthen material at the first time for each of the plurality of bands of interest; and apply the intensity of incident light at the earthen material at the first time for each of the plurality of bands of interest and the spectral imager data to determine a reflectance of the earthen material.

In yet a further example, an imaging method for earthen material includes: acquiring, at a first time, spectral imager data of an earthen material at an image location exposed to varying and uncontrolled illumination, the spectral imager data captured by a spectral imager directed at and focused on the image location and including the intensity of illumination reflected from the earthen material for each of a plurality of bands of interest; acquiring, at a second time, spectral imager data of a reference from a field of view of the spectral imager, the second time being different from the first time and the spectral imager data of the reference including the intensity for one or more reference wavelength bands; applying the spectral imager data of the reference to determine an intensity of the varying and uncontrolled illumination at the second time for the one or more reference wavelength bands; applying the intensity of the varying and uncontrolled illumination at the second time to infer an intensity of incident light at the earthen material at the first time for each of the plurality of bands of interest; and applying the intensity of incident light at the earthen material at the first time for each of the plurality of bands of interest and the spectral imager data to determine a reflectance of the earthen material.

In another example, an imaging system for earthen material includes: a support structure adjacent an image location exposed to varying and uncontrolled illumination; a spectral imager mounted to the support structure, the spectral imager directed in a first direction at the image location and operable to measure an intensity of illumination reflected from the earthen material at the image location for each of a plurality of wavelength bands of interest; and first and second reference devices directed in a second direction different from the first direction, the second reference device located below the first reference device to directly monitor the image location area for a shadow of the support structure, the first and second reference devices operable to sample the varying and uncontrolled illumination to which the image location is exposed for one or more reference wavelength bands at two different locations.

In another example, an imaging system for earthen material includes: a support structure adjacent an image location exposed to varying and uncontrolled illumination; a spectral imager mounted to the support structure, the spectral imager directed in a first direction at the image location and operable to measure an intensity of illumination reflected from the earthen material at the image location for each of a plurality of wavelength bands of interest; first and second reference devices directed in a second direction different from the first direction, the second reference device located below the first reference device to directly monitor the image location area for a shadow of the support structure, the first and second reference devices operable to sample the varying and uncontrolled illumination to which the image location is exposed for one or more reference wavelength bands at two different locations, and a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to: direct the spectral imager to capture spectral imager data of an earthen material at the image location, the spectral imager data including the intensity of illumination reflected from the earthen material for each of the plurality of bands of interest; receive the spectral imager data from the spectral imager; direct the first reference device to capture first illumination data from a sample of the varying and uncontrolled illumination sampled from the first reference device, the first illumination data including the intensity of the varying and uncontrolled illumination for the one or more reference wavelength bands at a first location; direct the second reference device to capture second illumination data from a sample of the varying and uncontrolled illumination sampled by the second reference device, the second illumination data including the intensity of the varying and uncontrolled illumination for the one or more reference wavelength bands at a second location; receive the first and second illumination data; apply the first and second illumination data to determine a difference in an intensity of incident light at the image location for the one or more reference wavelength bands; if the first and second illumination data are different, then apply the spectral imager data and the intensity of incident light affected by shadow cover to determine a reflectance of the earthen material for each of the plurality of bands of interest, otherwise apply the spectral imager data and the intensity of incident light associated with the first and second illumination data to determine a reflectance of the earthen material for each of the plurality of bands of interest.

In a further example, an imaging system for earthen material includes: a support structure adjacent an image location exposed to varying and uncontrolled illumination; a spectral imager mounted to the support structure, the spectral imager directed at and focused on the image location and operable to measure an intensity of illumination reflected from the image location for each of a plurality of bands of interest; a reference object in a field of view of the spectral imager beyond the image location, at least one reference device directed in a second direction different from the first direction, the at least one reference device operable to sample the varying and uncontrolled illumination to which the image location is exposed for one or more reference wavelength bands, and a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to: direct the spectral imager to capture spectral imager data of an earthen material at the image location, the spectral imager data including the intensity of illumination reflected from the earthen material for each of the plurality of bands of interest; receive the spectral imager data associated with the earthen material from the spectral imager; direct the spectral imager to capture spectral imager data of the reference object, the spectral imager data including the intensity of illumination reflected from the reference object for the one or more reference wavelength bands; receive the spectral imager data associated with the reference object from the spectral imager; direct the capture of illumination data from a sample of the varying and uncontrolled illumination sampled by the at least one reference device, the illumination data including the intensity of the varying and uncontrolled illumination for the one or more reference wavelength bands; receive the illumination data; identifying intensity variations among pixels associated with shadowing in the one or more reference wavelength bands captured in the spectral imager data associated with the reference object from the spectral imager; apply the illumination data as the intensity of incident light at the image location for each of the plurality of bands of interest; determine the percentage of intensity of incident light at the pixel locations associated with shadowing in the plurality of bands of interest captured in the spectral imager data associated with the reference object from the spectral imager; apply the spectral imager data and the intensity of incident light per pixel to determine a reflectance of the earthen material for each of the plurality of bands of interest, wherein those pixels associated with shadowing in the plurality of bands of interest captured in the spectral imager data associated with the reference object from the spectral imager as adjusted by the percentage of intensity of incident light.

### BRIEF DESCRIPTION OF THE DRAWINGS

The drawings included herewith are for illustrating various examples of articles, methods, and apparatuses of the present specification and are not intended to limit the scope of what is taught in any way. In the drawings:
Figure 1 is a perspective view of a first imaging system extending over a haul truck, according to an embodiment;
Figure 2 is a perspective view of the imaging system of Figure 1 extending over a conveyor belt;
Figure 3 is an end view of a second imaging system, according to an embodiment;
Figure 4 is an end view of a third imaging system, according to an embodiment;
Figure 5 is an end view of a fourth imaging system, according to an embodiment;
Figure 6 is an end view of a fifth imaging system, according to an embodiment;
Figure 7 is a flow chart of a first imaging method, according to an embodiment;
Figure 8 is an end view of a sixth imaging system, according to an embodiment;
Figure 9 is an end view of a seventh imaging system, according to an embodiment;
Figure 10 is an end view of an eighth imaging system, according to an embodiment;
Figure 11 is an end view of a ninth imaging system, according to an embodiment; and,
Figure 12 is a flow chart of a second imaging method, according to an embodiment.

### DETAILED DESCRIPTION

The present disclosure is directed to applications in which spectral information about a material is acquired while the material is exposed to varying and uncontrolled incident illumination. An intensity of the varying and uncontrolled incident illumination is measured directly or indirectly and used with the acquired spectral information to determine a reflectance of the material. The material may concurrently be exposed to controlled incident illumination (e.g., an artificial emission source providing a known amount of illumination intensity) and a varying and uncontrolled incident illumination (e.g., exposed to solar light). The intensity of illumination reflected from any object may be quantified as an amount or ratio from 0-1 or 0-100% of illumination reflected from an object to the total amount of illumination received by the object. The reflectance of the material may include accounting for both the varying and uncontrolled incident illumination and the controlled incident illumination. Accounting for the controlled incident illumination may include using known positional and emission characteristics of the artificial emission source to calculate the controlled incident illumination and/or using direct or indirect measurement of the controlled incident illumination.

### General Description of an Imaging System

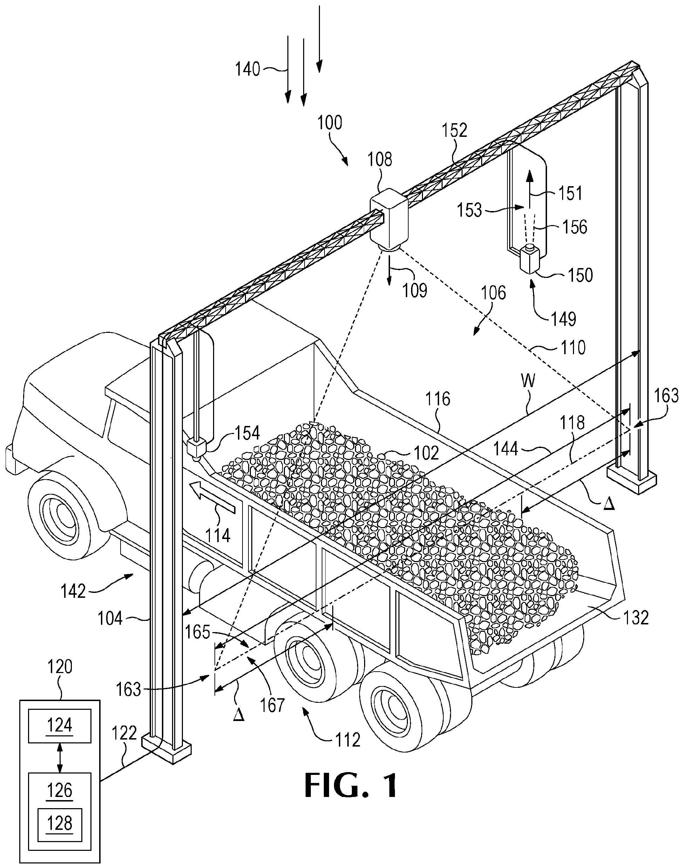

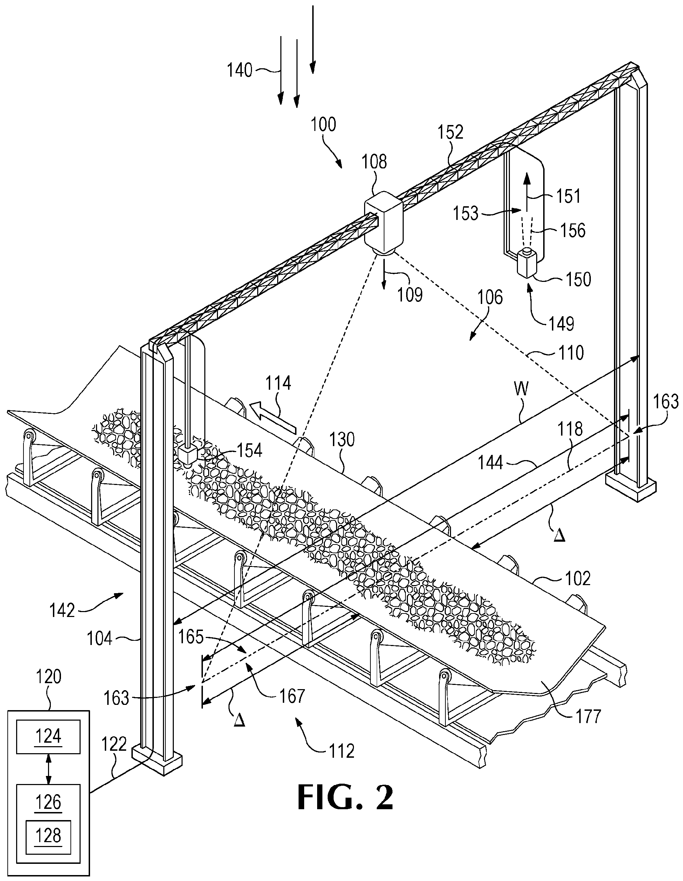

As exemplified in Figures 1 and 2, an imaging system 100 is provided to acquire spectral information about a material 102. The imaging system 100 includes a support structure 104 adjacent to an image location 106. The imaging system 100 also includes a spectral imager 108 mounted to the support structure 104. The spectral imager 108 is operable to capture intensity data for a plurality of discrete wavelength bands of illumination from the imager's field of view 110 to form an image. While a single spectral imager 108 is described, it will be appreciated that imaging system 100 may include a plurality of spectral imagers imaging a common image location or imaging a plurality of image locations. For example, the spectral imager 108 may be a set of spectral imagers located on either side of the support structure 104.

The spectral imager 108 is directed in a first direction 109 (e.g., downward) and at the image location 106 and arranged to measure an intensity of illumination reflected from the image location 106 for the plurality of discrete wavelength bands. As exemplified, the image location 106 is in a field of view 110 of the spectral imager 108. The field of view 110 is illustrated as diverging from the spectral imager 108 such that the entire pathway from side to side is covered. In other examples, the field of view 110 may be more narrowed with some deviation Δ to account for different material transport paths 112 and widths W through the structure 108. This may be accomplished with at least one lens in the spectral imager 108. When the material 102 is arranged at the image location 106, illumination reflected from the material 102 is able to be captured by the spectral imager 108. Mounting the spectral imager 108 to a support structure adjacent (e.g., above) the image location 106 allows the material 102 to be imaged by the spectral imager 108 as the material 102 passes through the image location 106.

In some examples, the spectral imager 108 is a hyperspectral camera. The spectral imager 108 may include more than one hyperspectral camera per unit. A hyperspectral camera captures intensity information for many wavelength bands of illumination for each individual pixel from the hyperspectral camera's field of view (e.g., 37 or more spectral bands). The hyperspectral camera may also capture intensity information for less discrete bands. The bands of a hyperspectral camera may be for consecutive wavelength ranges (i.e., not separated by unsampled portions of the spectrum). The information captured by a hyperspectral camera has a fine spectral resolution allowing for more accurate material identification. In some examples, the information captured has 1 to 10 nm resolutions. In some examples, the resolution is less than 10 nm for consecutive high resolution imagery. In some examples, the spectral imager is a line scanning hyperspectral camera (i.e., a push broom scanner). In the illustrated example, the spectral imager 108 is a line scanning hyperspectral camera arranged with the field of view 110 and image line 118 extending generally transverse to a direction of movement 114 of the material 102 along the material transport path 112. The spectral imager 108 defines the field of view 110 and image line 118 extending generally transverse to the direction of movement 114 may image strips of the material 102 as the material 102 travels along the material transport path 112. The image line 118 is preferably situated above the ground at a level of the material 102 to be measured. If multiple spectral imagers are used, then anything below a chosen level or distance from the imager 108 (e.g., anything below the image line 118) can be ignored as needed (e.g., the ground can be ignored).

As exemplified, the support structure 104 may extend over the image location 106. The material transport path 112 may extend under the support structure 104. The spectral imager 108 may be mounted to the support structure 104 looking down at the image location 106. The support structure 104 is illustrated as a U-shaped structure with the spectral imager 108 centrally located, but other configurations are possible. For example, the support structure may be L-shaped (e.g., a supporting column with a cantilevered support extending over the material transport path 112) with the spectral imager 108 located near an end of the structure (e.g., closer to the end of the cantilevered support than to the supporting column). Directing the spectral imager 108 downward (e.g., vertically downward or at an angle) at the image location allows a top surface of the material 102 to be imaged. The material 102 may be a loose pile of material, and the top surface may be the most exposed surface. As exemplified, the top surface of the material 102 may be imaged as the material 102 is carried along the material transport path 112. As exemplified, the field of view 110 of the spectral imager 108 may span the material transport path 112. In some examples, the imaging system includes a plurality of spectral imagers and the combined fields of view of the spectral imagers span the material transport path.

In some examples, the imaging system 100 also includes a controller 120. The controller 120 is communicatively coupled to the spectral imager 108 (e.g., via a wired and/or wireless network 122) to control operations of the spectral imager 108 and to receive imaging data from the spectral imager 108. In some examples, the controller 120 includes at least one processor 124 and at least one data storage device 126. The controller 120 may be distributed or a single machine. The controller 120 may be virtual or real. The controller 120 may be remote as illustrated or local.

The at least one data storage device 126 stores a set of computer-executable instructions 128 for operating the at least one processor 124. The at least one data storage device 126 may also store one or more schedule, such as any of the schedules described herein.

The set of instructions 128 include instructions to acquire from the spectral imager 108 a set of spectral imager data of the material 102 when the material 102 is at the image location. Acquiring spectral imager data from the spectral imager includes directing the spectral imager 108 to capture the spectral imager data and receiving the imaging data from the spectral imager. The spectral imager 108 may be operated in any suitable way to capture the spectral imager data, such as in accordance with a predetermined schedule or in response to a sensed acquisition criteria (e.g., sensing that a truck is driving through the image location). Imaging data captured by the spectral imager 108 may be captured only when the material 102 is in the image location 106 and/or may be processed after acquisition to isolate the spectral imager data of the material. The spectral imager data includes intensity data for each of a plurality of wavelength bands of interest. The bands of interest may be selected based on the application (e.g., infrared wavelengths), such as selected to correspond to bands in which a composition of interest would exhibit emission or absorption lines. Absorption and emission lines are not the only useful data, and knowing the amount of light reflected at each wavelength provides information regardless of whether there is an absorption line. In some examples, the bands of interest include many consecutive bands covering a broad range of wavelengths (e.g., hyperspectral imaging data). In some examples, the shape of a continuous spectrum is used to identify a composition of a material.

However, as discussed elsewhere herein, determining the reflectance of a material at wavelength bands of interest requires comparing acquired intensity data for the wavelength bands of interest to the intensity of light received by the material for the wavelength bands of interest.

The present disclosure is directed to determining the reflectance of the material when the material is exposed to uncontrolled and variable illumination 140. The image location 106 may be an outdoor location, e.g., exposed to variable and uncontrolled solar illumination. Reducing the need for a housing around the image location and associated spaces may facilitate movement of material to and from the image location to be imaged and/or reduce the cost of the imaging system.

In some examples, the uncontrolled and variable illumination 140 is incident upon an earthen material. The imaging system 100 may be an earthen material imaging system configured to identify earthen materials (e.g., storing spectral information for one or more known earthen materials). Identifying a material may include identifying a particular element or elements found in the material or, also or alternatively, determining one or more material properties (e.g., illite crystallonity index, moisture content, and the like). The earthen material 102 has a non-uniform surface texture and is composed of a plurality of constituents (e.g., different types or rocks and/or soils which may be piled together on, e.g., a conveyor belt or the bed of a truck). The imaging system 100 is arranged in a facility 142, which may be a mining facility.

For example, earthen material 102 may need to be moved rapidly through the image location 106, the imaging system 100 may need to accommodate large equipment (e.g., haul trucks) moving the earthen material through the image location 106, the mining activity may cause variations in incident illumination intensity (e.g., dust stirred up by mining operations changing the characteristics of incident solar illumination), and/or the transport of the material 102 may cause variations in incident illumination intensity (e.g., due to dust stirred up by transport vehicles).

The image location 106 may be along an earthen material transport path 112 extending from a mine to and through a processing facility. In some examples, the material transport path 112 is a haul truck route used by haul trucks 116 (Figure 1), and the image location is at the haul truck route. In some examples the image location is at a conveyor system transporting earthen materials, such as a conveyor belt 130 (Figure 2) extending from a mine to a processing facility or between zones of a processing facility. In some examples, the image location may be at a processing facility, such as at a crusher stage of a processing facility. The spectral imager may be directed at material being transported between processor stages, material being deposited into a stage (e.g., directed at material being dumped into the crusher stage) or material that is in the stage (e.g., directed at material in the crusher). The earthen material 102 may be loosely arranged on a support surface (e.g., a conveyor belt or haul truck bed) as it passes through the image location 106 to be imaged by the spectral imager 108. The support structure 104, spectral imager field of view 110, and/or image location 106 may be sized and positioned to accommodate the movement of the support surface on which the material 102 is arranged.

As illustrated in Figure 1, the image location 106 is at the haul truck route and the spectral imager 108 is arranged to image a top surface of earthen material 102 distributed into a bed 132 of a haul truck 116. For example, the earthen material 102 may be a pile of rocks and/or soil in the bed of the haul truck 116. The field of view 110 of the spectral imager 108 may have a width 144 (e.g., 15 feet). The field of view 110 of the spectral imager 108 may span at least a portion of the width of the haul truck bed 132. As in the illustrated example, the field of view 110 of the spectral imager 108 may span at least the width of the haul truck bed 132.

### Imaging System with Reference Devices Sampling Illumination

Referring to Figure 1, the imaging system 100 accounts for uncontrolled illumination using one or more reference device(s) 149. The reference device 149 is mounted to the support structure 104. The reference device may be located below and offset from the support structure 104, but other configurations, such as atop the structure 104 are envisioned. The reference device 149 is located above the image location 106 and directed in a second direction 151 different from the first direction 109. The second direction 151 may be generally opposite to the first direction 109. The second direction 151 may be static and unchanging. The reference device 149 includes a housing and a sensor 150 or an inlet end of an optical system 155, discussed further elsewhere herein, that is operable to sample the varying and uncontrolled illumination 140 (e.g., the sensor 150 measures the varying and uncontrolled illumination or the optical system 155 redirects the varying and uncontrolled illumination to the spectral imager 108) to which the image location 106 is exposed (e.g., varying due to factors such as cloud cover, location of the sun, shadow cover of the structure, and the like). The reference device 149 samples the illumination 140 at a sample location 153. The reference device 149 may sample the same band or bands as measured by the spectral imager 108 (i.e., an identical set of bands), or may sample a different band or bands (e.g., a band that is not measured by the spectral imager 108, a set of bands none of which are measured by the spectral imager, or a set of bands that includes one or more that are not measured by the spectral imager but may include one or more that are measured by the spectral imager). In some examples, reference device samples a different band or bands form the band or bands of interest measured by the spectral imager 108, and the intensity of the bands of interest can be estimated from the band or bands sampled by the reference device. The sample location 153 is spaced from the image location 106, but other configurations are possible. In some examples, the distance between the sample location 153 and the image location 106 is a predetermined difference to allow the distance to be taken into account when applying data from the sample to determine intensity of illumination at the image location 106.

The imaging system 100 may include a plurality of reference devices 149 to sample the varying and uncontrolled illumination 140 from a plurality of spaced-apart sample locations. The plurality of reference devices 149 may be spaced apart from one another. The plurality of reference devices 149 may include a reference device 149 on either side of the structure 104 such that the structure 104 does not affect the measurement. The plurality of reference devices 149 may be each aligned with a different portion of the field of view 110 and/or image line 118 of the spectral imager to provide a sample representative of the varying and uncontrolled illumination for that portion of the spectral imager data. A plurality of spaced-apart reference devices 149 increases the accuracy of accounting for different illuminations across the field of view and/or image line of the spectral imager. For example, the plurality of reference devices 149 may include a reference device 149 corresponding to each pixel of an image acquired by the spectral imager 108, or a reference device 149 for each of a set of adjoining pixels (e.g., a square block of four or nine pixels or a linear strip of pixels). It will be appreciated that various techniques may be used to apply data from samples to pixels of an image captured by the spectral imager 108 when there are less reference devices 149 than pixels, such as linear interpolation. Where the spectral imager images a line, the reference devices 149 may be arranged in a line. The line of reference devices 149 may extend generally parallel to the image line 118 of the spectral imager 108.

As illustrated, the imaging system 100 may include an artificial illumination source 154 directed at the image location 106 from the same side of the image location 106 as the spectral imager 108. For example, the artificial illumination source 154 and the spectral imager 108 may both be generally above the image location 106 and directed downwardly at the image location 106. The artificial illumination sources 154 may be mounted to the support structure 104. In other examples, the artificial illumination source is a component of the spectral imager 108. The artificial illumination source 154 is arranged to provide controlled illumination to the image location 106. As exemplified, the controller 120 may be communicatively coupled to the source 154 (e.g., via network 122) to control operation of the source 154 (e.g., according to a schedule or in response to an input such as a motion detection).

In some examples, the controller is operably coupled to the at least one artificial light source to control when the at least one artificial light source is on and when the at least one artificial light source is off. The controller may be operable to switch the at least one artificial light source between off and on according to a schedule stored on the at least one data storage device. The controller may be operable to switch the at least one artificial light source on in response to detection of a shadow at the image location (e.g., detection by a discrete shadow detection sensor). In some examples, the artificial light source is always on because the mine site location is always affected by low light conditions. In some examples, the controller may be operable to switch the at least one artificial light source on in response to motion detected (e.g., detecting a quantity of the material at or approaching the image location 106).

The emission characteristics and the positional characteristics (e.g., angle and spacing relative to the image location) of the illumination source 154 are known (e.g., stored on the at least one data storage device 126), and the expected intensity of incident illumination at the image location for each of the bands of interest may be calculated. In some examples, the controller 120 (e.g., the at least one data storage device 126) stores a constant scaling factor and/or the emission characteristics and the positional characteristics for the controlled illumination, and accounting for the controlled illumination includes applying the scaling factor and/or calculating the intensity of controlled illumination received at the image location 106 from the artificial light source. Calculating an intensity may include using the inverse square law, calculating the decrease in intensity of light as a function of distance traveled at a squared rate. If the original light intensity and distance traveled is known, the reduction in intensity can be determined.

In some examples, the set of instructions 128 includes instructions to capture illumination data from samples of the varying and uncontrolled illumination 140 sampled by the reference device 149. The set of instructions 128 also includes instructions to receive the illumination data.

The reference device 149 may be positioned such that it is not exposed to the controlled illumination from the artificial light sources 154. For example, the artificial illumination source 154 may be spaced from the reference device 149, directed away from the reference device 149, and/or positioned lower than the reference device 149 and/or closer to the image location 106 than the refence device 149. Where the reference device 149 is not exposed to the controlled illumination, the illumination data sampled by the reference device does not include the controlled illumination. This may permit the calculated value of the controlled illumination to be added to the sampled illumination data without including the controlled illumination intensity more than once. In some applications it may be preferable to use the calculated value of the controlled illumination rather than the measured value. However, the reference device 149 may alternatively be exposed to the controlled illumination, in which case the measured controlled illumination may be used instead of, or in addition to, stored information about the controlled illumination.

Once the intensity of incident light at the image location for each of the plurality of bands of interest is determined, the reflectance of the material may be determined using the intensity of incident light and the spectral imager data captured by the spectral imager (e.g., this may include taking into account a percentage of the varying and uncontrolled illumination at certain pixel locations that are shadowed). The set of instructions 128 includes instructions to apply the spectral imager data and the determined intensity of incident light to determine a reflectance of the earthen material for each of the plurality of bands of interest. The reflectance for a particular band of interest may be, e.g., a value between zero and one indicating the fraction of incident light that was reflected for that band. The spectral imager data and the illumination data used to determine the intensity of the incident light may be acquired substantially simultaneously (e.g., within five second, within two seconds, or within one second) to provide for accurate calculations. However, it will be appreciated that the spectral imager data may also, or alternatively, be used with illumination data that was acquired before and/or after the spectral imager data.

When the imaging data captured by the spectral imager 108 is from the material 102, the intensity of the imaging data for each of the plurality of bands of interest is able to provide the reflectance of the material for the plurality of bands of interest on a per pixel basis. The instructions 128 may also include instructions to compare the determined reflectance of the material 102 to information for known materials to determine a composition of the material. Determining a composition may include identifying a particular element or elements found in the material or, also or alternatively, determining one or more material properties (e.g., illite crystallonity index, hardness, moisture content, and the like). Where the set of instructions 128 includes instructions to compare the reflectance of the material to information for known materials, the controller 120 (e.g., the data storage device) stores reflectance information for known materials for comparison, such as in the form of a library of information or a machine learned model.

The set of instructions 128 may include instructions to repeat one or more acquisition steps and/or application steps for different materials (e.g., different loads of mining material) and/or different images of the same material 102. In some examples, a determined intensity of incident light at the image location is used for more than one set of spectral imager data taken by the spectral imager, such as for a series of sequential images taken by the spectral imager. The set of instructions 128 may include instructions to acquire a new set of illumination data from the at least one reference sensor and apply the new set of illumination data to determine the intensity of incident light at the image location according to a schedule (e.g., spaced by a predetermined period of time or a predetermined number of times a new set of spectral imager data is taken) or in response to an input, such as sensing a change in solar illumination.

The illustrated reference device 149 includes a sensor 150 to sample the uncontrolled and variable illumination by measuring the uncontrolled and variable illumination. In embodiments in which the reference device 149 is an active device (i.e., performing steps in response to instructions, such as a sensor capturing imaging data in response to an instruction) the controller 120 is communicatively coupled to the reference device 149 (e.g., via a wired and/or wireless network 122) to control operations of the reference device 149 and to receive data from the reference device 149. The reference device 149 is directed away from the image location 106 (e.g., with a reference sensor field of view 156 that does not intersect the image location 106 or preferably the structure 104) and arranged to measure an intensity of the variable and uncontrolled illumination 140 for each of a plurality of wavelength bands of interest. A reference device 149 may be, e.g., a spectrometer or a linear array with a set of filters. In some examples, the reference device 149 is directed in a direction that is generally opposite to the direction in which the spectral imager 108 is directed (e.g., upward when the spectral imager 108 is directed downward). In some examples, the reference device 149 is generally directed at the sky above the image location 106.

As exemplified in Figure 1, the reference device 149 may be arranged in-line with a portion of the support structure and the image location, such that if a shadow is cast by the portion of the support structure on the image location it is experienced by (e.g., cast on) the reference device as well. Partial or complete shadowing can alter the amount of illumination that is reaching the surfaces of interest and impact the extent of the reflection. This can create complications in calculating the reflectance, particularly if only some pixels of the camera are aligned with the shadowed regions and other pixels are not.

As exemplified, the spectral imager 108 may be mounted to a portion 152 of the support structure (e.g., an arm or truss of the support structure 104) that is above the image location 106, and the reference device 149 may be mounted between the portion 152 of the support structure 104 to which the spectral imager 108 is mounted and the image location 106. The sample location 153 may be between the portion 152 and the image location 106.

Where the image location 106 is generally directly below a portion of the support structure (e.g., a support arm or truss), then the reference device 149 may also be mounted generally directly below the portion of the support structure 104 (e.g., the portion 152). Where the image location 106 is offset from a position directly below (e.g., shifted backwards relative to movement direction 114, as exemplified, due to the spectral imager 108 being angled relative to the vertical), the reference device 149 may also be offset from being directly below such that the reference device, the image location, and the portion of the support structure casting a shadow are all in line with one another. The reference device 149 may be directed at the portion of the support structure casting a shadow to experience the shadow cast by the portion of the support structure.

Referring still to Figure 1, the set of instructions 128 may further include computer-executable instructions to acquire illumination data from the reference device 149 (e.g., the sensor 150). Acquiring illumination data includes directing the reference device to capture illumination data and receiving the illumination data from the reference device. The illumination data includes the intensity of the variable and uncontrolled illumination 140 for each of the plurality of wavelength bands of interest. The instructions 128 may include instructions to acquire illumination data regularly, such as according to a predetermined schedule. The predetermined schedule may be set based on, e.g., regular intervals of time (e.g., once ever second, or once every minute) or regular intervals of (e.g., every time imaging data is acquired from the spectral imager, or every third time imaging data is acquired from the spectral imager). The scheduler may take into effect known time periods where the sun will cause a shadow on the structure 104 to obscure the imaging area 106. The artificial illumination device 154 may also be on a scheduled time to turn on when this shadow effect is known to happen during the day time hours or may turn on the artificial illumination device when a shadow (e.g., cloud coverage) is detected.

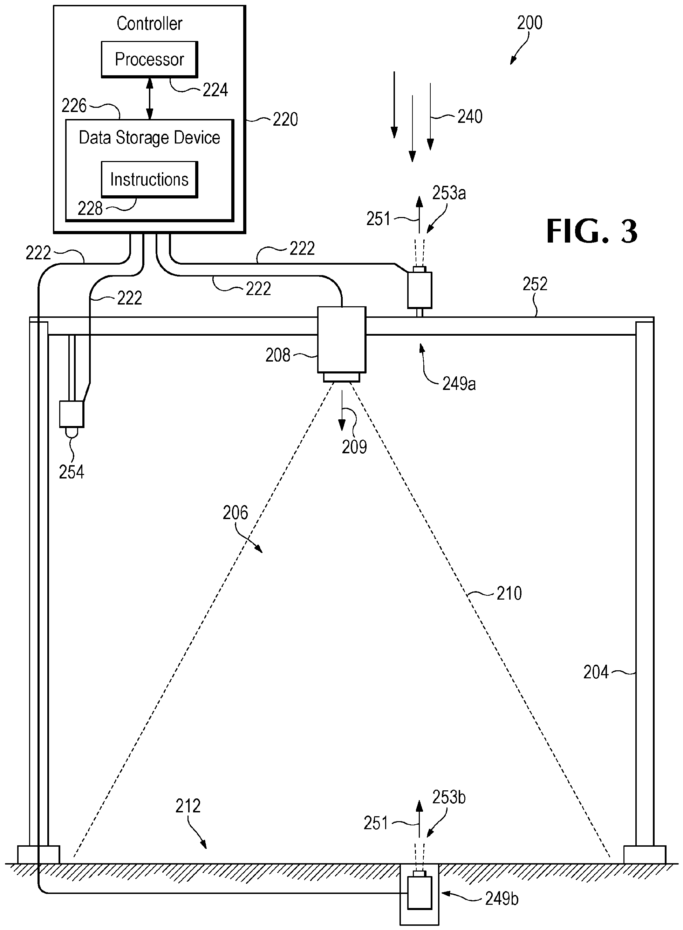

Referring to Figure 3, an imaging system 200 is illustrated that is substantially similar to the imaging system 100 of Figs. 1-2 with the noted exceptions, and like components are indicated by like reference numbers incremented by 100. As illustrated in Figure 3, the imaging system 200 may include a first reference device and a second reference device located below the first reference device to directly monitor the image location area for a shadow of the support structure.

As illustrated in Figure 3, a spectral imager 208 is secured to a support structure 204 above a material transport path 212. The spectral imager 208 has a field of view 210 directed in first direction 209 (e.g., downward), such as towards a haul truck 216 traveling along the material transport path 212. The spectral imager 208 may be a push broom scanner with an imaging line 218 at an image location 206 arranged to image material carried along the path 212. The system 200 may include artificial light source 254 to illuminate the image location 206.

The imaging system 200 includes a first reference device 249a and a second reference device 249b. The first and second reference devices 249a, 249b are directed in a second direction 251 different from the first direction 209. The second reference device 249b is located below the first reference device 249a to directly monitor the image location area 206 for a shadow of the support structure 204. The first and second reference devices 249a, 249b are operable to sample the varying and uncontrolled illumination to which the image location is exposed for each of the plurality of wavelength bands of interest at two different locations, the first reference device 249a sampling at first sampling location 253a and the second reference device 249b sampling at second sampling location 253b.

A controller 220 controls operations and/or receives information from the spectral imager 208 and/or the reference devices 249a, 249b (e.g., communicatively coupled via a communications network 222). In some examples, the controller 220 includes at least one processor 224 and at least one data storage device 226. The at least one data storage device 226 stores a set of computer-executable instructions 228 for operating the at least one processor 224.

The set of instructions 228 include instructions to direct the first reference device 249a to capture first illumination data from a sample of the varying and uncontrolled illumination 240 sampled from the first reference device 249a. The first illumination data includes the intensity of the varying and uncontrolled illumination 240 for each of the plurality of bands of interest at the first location 253a. The set of instructions 228 also include instructions to direct the second reference device 249b to capture second illumination data from a sample of the varying and uncontrolled illumination 240 sampled by the second reference device 249b. The second illumination data including the intensity of the varying and uncontrolled illumination 240 for each of the plurality of bands of interest at a second location 253b.

The set of instructions 228 include instructions to receive the first and second illumination data, and apply the first and second illumination data to determine a difference in an intensity of incident light at the image location for each of the plurality of bands of interest. The set of instructions 228 include instructions to, if the first and second illumination data are different, apply the spectral imager data and the intensity of incident light affected by shadow cover (e.g., from reference device 249b) to determine a reflectance of the earthen material for each of the plurality of bands of interest. The set of instructions 228 also include instructions to, if the first and second illumination data are not different (e.g., are the same), apply the spectral imager data and the intensity of incident light associated with the first and second illumination data to determine a reflectance of the earthen material for each of the plurality of bands of interest.

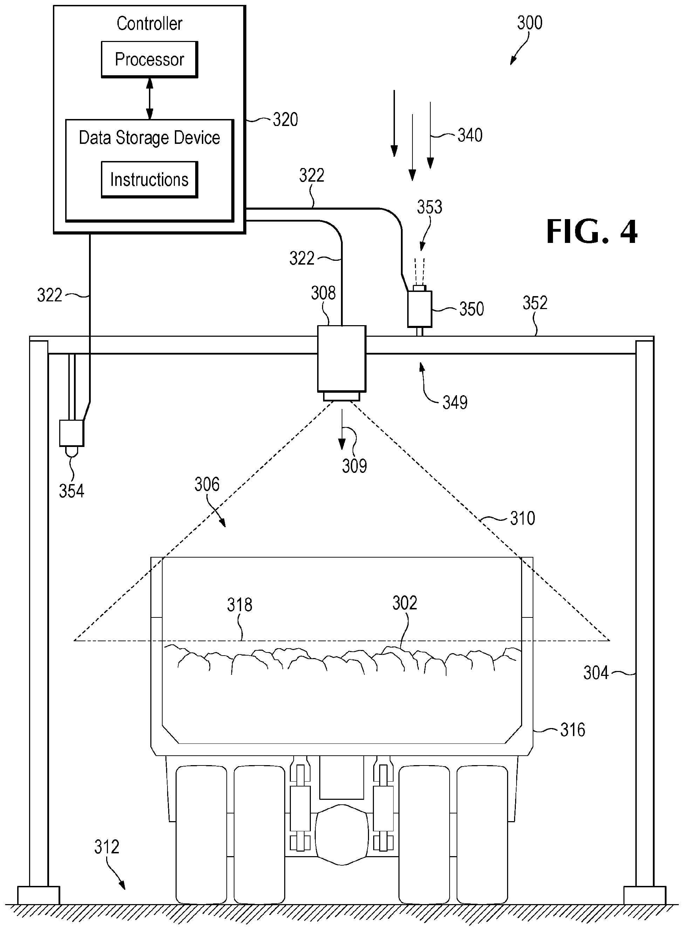

Referring to Figure 4, an imaging system 300 is illustrated that is substantially similar to the imaging system 100 of Figs. 1-2 with the noted exceptions, and like components are indicated by like reference numbers incremented by 200. As illustrated in Figure 3, the reference device 349 may be above the portion of the support structure that holds the spectral imager. In some examples, the reference device is not arranged to experience the same shadowing from the support structure as the image location, such as because the support structure is shaped or positioned such that no structure-generated shadowing is expected to occur at locations of importance. This may keep the reference device out of the way of transportation equipment (e.g., haul trucks) and/or allow for a smaller support structure.

As illustrated in Figure 4, a spectral imager 308 is secured to a support structure 304 above a material transport path 312. The spectral imager 308 has a field of view 310 directed in direction 309 (e.g., downward), such as towards a haul truck 316 traveling along the material transport path 312. The spectral imager 308 may be a push broom scanner with an imaging line 318 at an image location 06 arranged to image material 302 carried along the path 312. The system 300 may include artificial light source 354 to illuminate the image location 306.

A reference device 349 may be secured to the support structure 304. The reference device 349 may include a sensor 350. The reference device 349 that is arranged at a position in which a portion 352 of a support structure 304 is between the reference device 349 and/or the sample location 353 and an image location 306. The reference device 349 may be arranged such that no portion of the support structure is further from the image location 306 than the reference device 349 and in line with the reference device 349 and the image location 306. As exemplified, the reference device 349 may be above a portion 352 of the support structure 304 that extends over the image location 306, but other configurations are possible.

A controller 320 controls operations and/or receives information from the spectral imager 308 and/or the reference device 349 (e.g., communicatively coupled via a communications network 322).

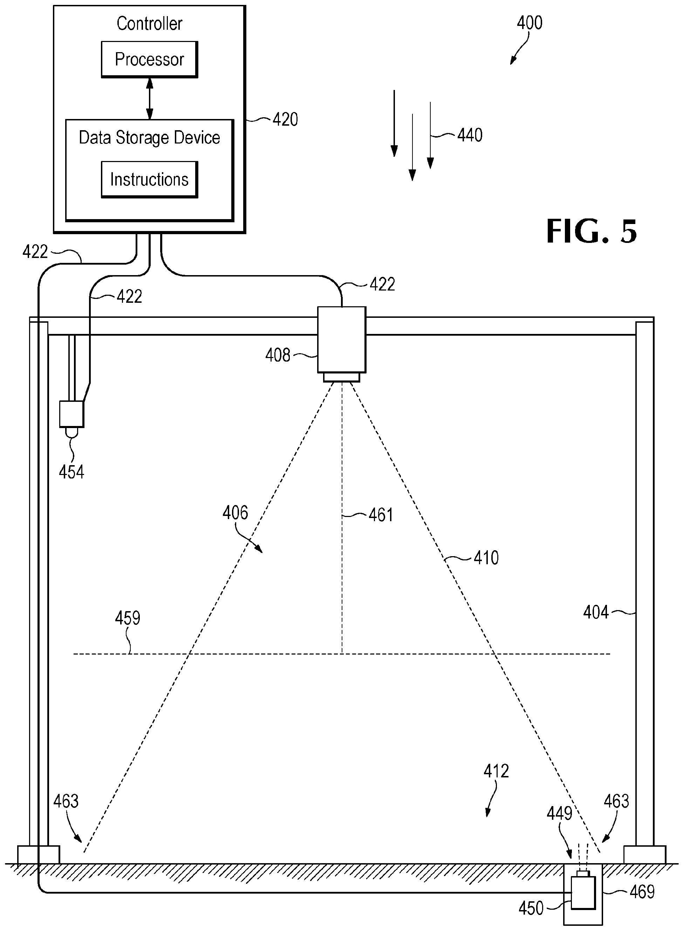

Referring to Figure 5, an imaging system 400 is illustrated that is substantially similar to the imaging system 100 of Figs. 1-2 with the noted exceptions, and like components are indicated by like reference numbers incremented by 300. As illustrated in Figure 5, the reference device may be across the image location from the spectral imager. This may allow the reference device to experience shadowing from the support structure. This may also, or alternatively, allow the reference device to be kept out of the way of transportation equipment and/or allow for a smaller support structure.

As illustrated in Figure 5, a spectral imager 408 is secured to a support structure 404 above a material transport path 412. The spectral imager 408 has a field of view 410 directed in direction 409 (e.g., downward), such as towards a haul truck 416 traveling along the material transport path 412. The spectral imager 408 may be a push broom scanner with an imaging line 418 at an image location 406 arranged to image material 402 carried along the path 412. The system 400 may include artificial light source 454 to illuminate the image location 406.

A reference device 449 may be positioned adjacent the image location 406. The reference device 449 may include a sensor 450. The reference device 449 is illustrated as not mounted to the support structure 404. A material movement plane 459 is a plane extending through the image location 406 and generally perpendicular to a straight line 461 between the spectral imager 408 to a center of the image location 406. The reference device 449 may be positioned across the material movement plane 459 from the spectral imager 408. The reference device 449 may be in a field of view 410 of the spectral imager 408 (e.g., beyond the image location 406) as illustrated or outside the field of view 410, such as arranged to one or both lateral sides 463 and/or to the forward and/or backward sides (illustrated as sides 165, 167 shown in Figure 1). The reference device 449 may be outside the material transport path 412 so that the transport system (e.g., haul truck 416) does not obstruct the reference device 449). The reference devices 449 may be below a material transport path 412, such as within a protective housing 469 set in the ground. In some examples, a top of the protective housing may form part of the haul truck route 412.

A controller 420 controls operations and/or receives information from the spectral imager 408 and/or the reference device 449 (e.g., communicatively coupled via a communications network 422).

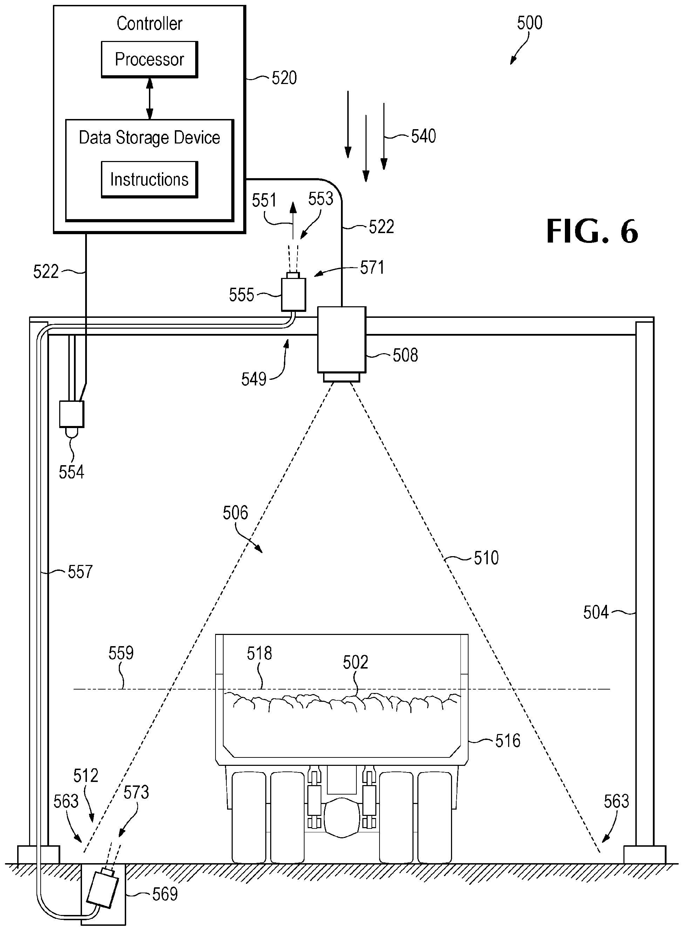

Referring to Figure 6, an imaging system 500 is illustrated that is substantially similar to the imaging system 100 of Figs. 1-2 with the noted exceptions, and like components are indicated by like reference numbers incremented by 400. As illustrated in Figure 6, the reference device may be an inlet end of an optical system that redirects the sampled varying and uncontrolled illumination to the spectral imager.

As illustrated in Figure 6, a spectral imager 508 is secured to a support structure 504 above a material transport path 512. The spectral imager 508 has a field of view 510 directed in direction 509 (e.g., downward), such as towards a haul truck 516 traveling along the material transport path 512. The spectral imager 508 may be a push broom scanner with an imaging line 518 at an image location 506 arranged to image material 502 carried along the path 512. The system 500 may include artificial light source 554 to illuminate the image location 506.

A reference device 549 is arranged adjacent the image location 506. The reference device is illustrated as a path inlet end 571 of an optical system 555. However, in some examples the optical system 555 may include one or more mirrors or other reflective surfaces to redirect a sample of the varying and uncontrolled illumination 540 toward the spectral imager 508. The optical system 555 may be used with or without the artificial illumination devices 554.

The illustrated optical system 555 forms an optical path 557. The optical path 557 may be a fiber optic cable. The fiber optic cable may be used as a downwelling radiance sensor. The optical path 557 may redirect a sample of the varying and uncontrolled illumination 540 from the sample location 553 towards the spectral imager 508 to be captured by the spectral imager 508. The optical path 557 extends between the path inlet end 571 at the sample location 553 and a path outlet end 573. The path inlet end 571 is directed in a second direction 551. The path outlet end 573 is directed towards the spectral imager 508. The optical path 557 redirects the sample sampled by the inlet end 571 directed in the second direction 551 to the spectral imager 508 facing the first direction 509. In some examples, the reference device 549 includes a plurality of inlet ends 571 with a common outlet end 573. For example, a plurality of paths 557 may have a common outlet end 573, such as if a plurality of fiber optic tubes joined together to carry light to a common outlet end 573. In some examples, an outlet end is modulated. A shared outlet end may output a sample from one input end at a time (e.g., using gates to block other inputs). The output may change at a high frequency, such as at least several times every second.

The path inlet end 571 may be in any suitable location, such as any of the locations described for the reference devices outlined above. For example, the inlet end 571 may be above the support structure 504, below the support structure 504, or across the material movement plane from the spectral imager 508. The inlet end 571 may be secured to the support structure 504 or in the ground below the transport path 512. The outlet end 573 is in the field of view 510. The outlet end 573 may be across the material movement plane 559 from the spectral imager 508, or may be on the same side of the material movement plane 559 as the spectral imager 508. For example, the outlet end 573 may be under the material transport path 512 (e.g., in a protective housing 569), or suspended below the spectral imager 508 but above the image line 518.

As illustrated, where the outlet end 573 is across the material movement plane 559 from the spectral imager 508, the outlet end 573 may be arranged such that a line of sight between the imager 508 and the outlet end 573 is not blocked by the transport system carrying the material 502 (e.g., not blocked by the truck 516). The outlet end 573 may be arranged at one of the lateral edges 563 of the field of view 510. One or more outlet ends 573 may be arranged at each lateral edge 563 of the field of view 510.

This may allow the outlet end(s) 573 to be visible past a transport surface carrying the material when the material is moving through the field of view 510 if the outlet end(s) 573 are beyond the material 502, or allow the outlet end(s) 573 to be to one side of the material 502 in the image so as not to obscure the material 502 if the end(s) 573 are closer to the imager 508 than the material 502.

However, the end 573 may alternatively be closer to the center of the field of view 510. Where the end 573 would be obscured by the transport system (e.g., the truck 516), the spectral imager 508 may capture imaging data of the outlet end 573 at a different time from when capturing imaging data of material 502 carried by the transport system.

In some examples, the capture of the illumination data includes directing the spectral imager to capture the illumination data from the sample provided by the outlet end(s). In some examples, a sample carried to the outlet end is captured by the spectral imager when the spectral imager captures an image of the material. For example, a pixel or a subset of pixels of the image of the material may capture the sample illumination. However, in other examples the illumination data is captured by the spectral imager at a different time than the spectral imager data.

In examples, the set of instructions includes instructions to apply the illumination data to determine an intensity of incident light at the image location for each of the plurality of bands of interest. Applying the illumination data to determine an intensity of incident light at the image location may involve equating the determined intensity of incident light at the image location to the illumination data or equating the determined intensity of incident light to the illumination data as modified by a predetermined equation or scalar value. However, in some examples, applying the illumination data to determine an intensity of incident light at the image location includes using more than one set of illumination data from the reference sensor (e.g., sequentially captured sets of illumination data separated by a predetermined period of time) and/or accounting for one or more additional light sources.

The additional light sources provide a known intensity of illumination at the image location for the bands of interest. The known intensity may be a measured intensity (e.g., for constant illumination) or a calculated intensity (e.g., for controlled illumination such as from artificial light sources having known emission characteristics and positional characteristics). It will be appreciated that controlled illumination may be constant or may vary in a predicted way, allowing the imaging system to account for the controlled illumination via calculations from predetermined information rather than from regular measurement.

A controller 520 controls operations and/or receives information from the spectral imager 508 (e.g., communicatively coupled via a communications network 522). In embodiments in which the reference device 549 is a passive device, the controller 520 may not be communicatively coupled to the reference device 549. However, in some examples, the controller 520 may be communicatively coupled to the reference device 549 even when the reference device 549 is an optical system, such as to adjust the positioning of reflective surfaces or modulate the output of a shared outlet end of an optical path.

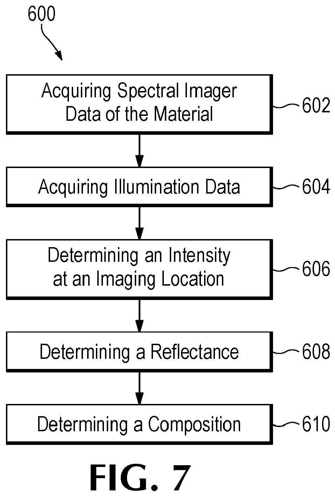

Referring to Figure 7, illustrated is an imaging method 600.The imaging method 600 may be a computer implemented method. Method 600 may be carried out by an imaging system having one or more reference device, such as any of the imaging systems described herein. Method 600 may be carried out by a portion of an imaging system disclosed herein, such as by the controller.

The imaging method 600 includes, at step 602, acquiring spectral imager data of a material at an image location exposed to varying and uncontrolled illumination and/or controlled illumination. The spectral imager data includes the intensity of illumination reflected from the material for each of a plurality of bands of interest. The material may be earthen material.

At step 604, the method 600 includes acquiring illumination data from the varying and uncontrolled illumination source sampled by one or more reference devices mounted adjacent the image location and directed in a different direction from that of the device used to acquire the spectral imager data. The illumination data includes the intensity of the varying and uncontrolled illumination for each of the plurality of bands of interest.

At step 606, method 600 includes applying the illumination data to determine an intensity of incident light at the image location for each of the plurality of bands of interest. Step 606 includes accounting for the varying and uncontrolled illumination, and may include accounting for controlled illumination.

Method 600 includes, at step 608, applying the spectral imager data and the intensity of incident light to determine a reflectance of the material for each of the plurality of bands of interest. At step 610, the method 600 may include comparing the reflectance of the material to stored reflectance data for known materials to determine an estimation of the composition of the material based on the surface level sampling. Determining a composition may include identifying a particular element or elements found in the material or, also or alternatively, determining one or more material properties (e.g., illite crystallonity index, hardness, moisture content, and the like). Step 610 may include comparing the reflectance of the material to a library of reflectance data for known materials or inputting the reflectance of the material to a machine learned model.

Method 600 or specific steps thereof may be repeated for different materials (e.g., different loads of mining material) and/or different images of a material. In some examples, an intensity of incident light at the image location determined at step 606 is used for more than one set of spectral imager data acquired at step 602, such as for a series of sequential images taken by the spectral imager, and steps 604 and 606 may be performed less frequently than steps 602, 608, and 610. In some examples, steps 604 and 606 may be performed according to a schedule (e.g., spaced by a predetermined period of time or a predetermined number of instances of step 602) or in response to an input, such as sensing a change in solar illumination.

### Imaging System Comparing Data Captured at Different Times

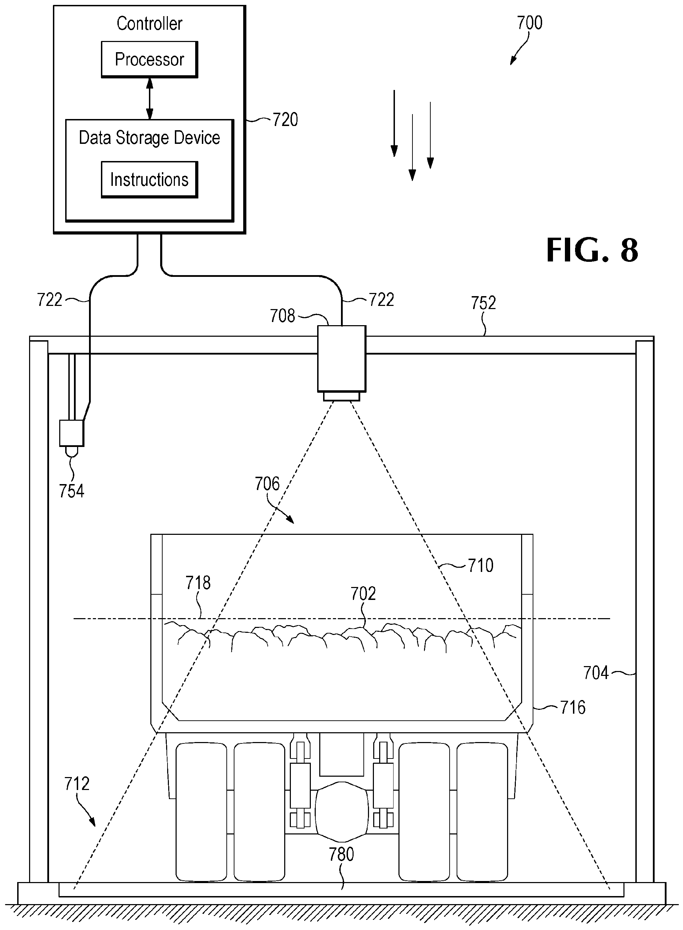

Turning now to Figures 8 to 12, in some examples the imaging system accounts for uncontrolled illumination using spectral imager data captured at a first time and illumination data captured at a second time that is different from the first time.

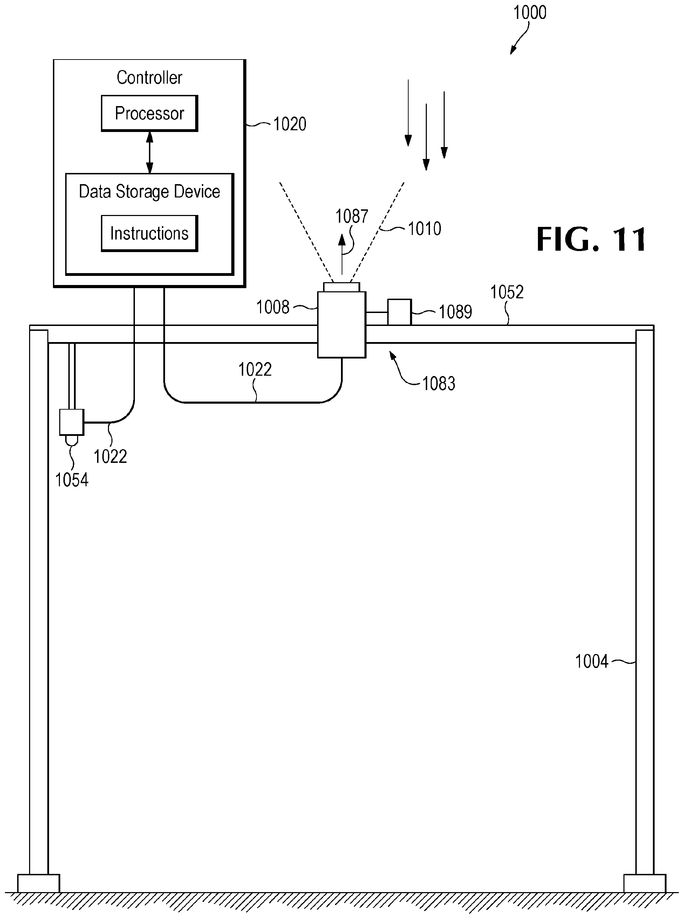

In examples in which illumination data from a second time is used, the set of instructions includes instructions to the spectral imager to acquire spectral imager data of the reference at a second time that is different from a first time at which the spectral imager data is acquired. This spectral imager data of the reference may be, e.g., of a reference object in the field of view of the imager (e.g., Figure 8 to 10) or data captured when the imager is in a different position (e.g., Figure 11).

Referring to Figure 8, an imaging system 700 is illustrated that is substantially similar to the imaging system 100 of Figs. 1-2 with the noted exceptions, and like components are indicated by like reference numbers incremented by 600. As illustrated in Figure 8, in some examples the imaging system accounts for uncontrolled illumination using a reference object arranged in the field of view of the spectral imager in lieu of a reference device or with a reference device.

As exemplified in Figure 8, a spectral imager 708 is secured to a support structure 704 above a material transport path 712. The spectral imager 708 has a field of view 710. The spectral imager 708 is directed in a first direction 709. The spectral imager 708 may be a push broom scanner with an imaging line 718 at an image location 706 arranged to image material 702 carried along the path 712 (e.g., by haul truck 716). The system 700 may include artificial light source 754 to illuminate the image location 706.

As exemplified, the reference object 780 may extend across substantially all of the field of view 710 or at least as much of the field of view 710 as the material 702. The reference object 780 may be used instead of the reference device, although it will be appreciated that an imaging system may include both reference object 780 and reference device. As discussed elsewhere herein, the set of instructions includes instructions to acquire spectral imager data of the material at the image location 706 using the spectral imager 708. In examples in which the imaging system 700 includes the reference object 780, the set of instructions 728 includes instructions to acquire spectral imager data of the reference object 780. The spectral imager data of the reference includes the intensity of illumination reflected from the reference object for each of the plurality of bands of interest (e.g. 3 bands to include 1400-1500nm, 2100-2200nm, and 2200-2300nm). The bands of interest may include bands that aid in determining the likelihood that a vehicle and/or a load container with earthen material is within the field of view. The vehicle may be a haul truck, a railcar, a barge, a trolley, a LHD vehicle, or a mining skip. In another example, other sensors such as cameras are used in combination with processors to determine the likelihood that a vehicle is within the field of view.

In some examples in which the reference object 780 is used, a controller 720 (e.g., the at least one data storage device) stores predetermined reflectance characteristics of the reference object 780. The reflectance of the reference object is a function of the wavelength of light received by the reference object and the reflectance characteristics of the reference object. The reference object 780 may consists of a Lambertian surface with a known reflectance across each spectral wavelength. Therefore, incident light is reflected equally in all directions regardless of the incidence angle, allowing for an accurate measurement of incident radiance. In other examples, the reference object 780 surface is non-Lambertian. In examples in which the reference object is used, the set of instructions also includes instructions to apply the spectral imager data of the reference and the reflectance characteristics of the reference object 780 to determine the intensity of incident light at the reference object at the time the spectral imager data of the reference was captured.

In another example, the computer instructions may also include gathering parameters, readings, or data from atmospheric sensors, irradiance sensors, photometers, temperature sensors, the date, the time, the geographical location of the spectral imager and the material, and the like to determine incident radiance and correct for atmospheric effects. For example, concurrent to the measurement of incident radiance using the reference object, the method may also estimate the incident radiance using a radiative transfer model. Software such as 6S or MODTRAN radiative transfer models could use the gathered parameters, readings, data, or extrapolated data to estimate the incident light at the spectral imager location. In another example, the instructions may include utilizing both the output from the radiative transfer model and the spectral imager data of the reference object 780. The reference object 780 offers a high-fidelity baseline reading for incident radiance. However, constant usage of the reference object 780 may be difficult to achieve (e.g. environmental factors). The radiative transfer model provides less accurate results. After the initial measurement (TO), if the reference object is no longer available to measure incident radiance, then the radiative transfer model will be available for subsequent estimation of incident radiance. In order to mitigate the reduction in accuracy of the radiative transfer model, a dynamic offset correction method may be utilized. For example, at each timestep (T1 and onwards), the change between the current and previous estimates (Tx-1 - Tx) from the radiative transfer model is computed. The delta is then used to adjust the previous incident radiance to get the current incident radiance. This method has the advantage over other methods that just use a radiative transfer model alone, as the reference object provides a more accurate baseline that can be adjusted temporally using the radiative transfer model when the reference object 780 is unavailable. It also has the advantage over methods that use a reference object only as this correction step can be performed in real-time without prior preparation after TO (e.g. there is no need to place and maintain a reference object in the scene).

In some examples in which the reference object is used, the reference object and the material are not imaged simultaneously. In examples in which the reference object is used, a spectral measurement of the material is taken at a first time and a second spectral measurement of the reference object is taken at a second time. The second time is different (e.g., before or after) from the first time. The spectral imager 708 may be in the same position at the first time and at the second time. The first time may be subsequent to the second time (e.g., to capture spectral imager data of the reference prior to changes to illumination caused by the movement of the material on the pathway, such as dust stirred up by passing trucks). For example, the first time may be when the material is at the imaging location and the second time may be when the material is not at the imaging location. For example, the first time may be when a haul truck loaded with material 702 is at the imaging location, and the second time may be before the loaded haul truck arrives at the imaging location, e.g., between haul trucks. The second time may be spaced from the first time or may be directly after or before by, e.g., at least five seconds, at least twenty seconds, less than an hour, less than thirty minutes, and/or between thirty seconds and twenty minutes. The spectral imager 708 may capture images according to, e.g., a schedule (e.g., regularly every occasion a predetermined time elapses, such as the time between the first time and the second time), or in response to input (e.g., sensor input, such as indicating the approach and/or arrival of a truck), or by checking for a likelihood of material 702 is within the field of view.

As exemplified, the reference object 780 may be arranged in the field of view 710 of the spectral imager 708 beyond the image location 706 on which the spectral imager 708 is focused. When the material 702 is at the image location 706, the reference object 780 may be partially or fully obscured by the material 702 or a conveyance carrying the material.

As discussed elsewhere herein, the imaging system may be an earthen material imaging system and may be operating in a mining facility. The material may be carried along a material transportation path that extends through the image location. In an active mining facility, it is difficult to simultaneously measure a reference object and a material across the entirety of the field of view while the material moves through the image location. Accordingly, the material and the reference object are sequentially imaged.

The reference object 780 is an object for which the reflectance characteristics are known. The reference object may be, e.g., the support surface on which the material rests at the image location or another face of a conveyance used to carry the material into the image location 706. For example, the reference object 780 may be an upward facing surface 177 of a conveyor belt 130 that carries the material (Figure 2), or an upward facing surface of the bed 132 of the truck 116 (Figure 1) or another surface of the truck 116. In some embodiments, the upward facing surface of the convenance (e.g., the upward facing surface 177 of the conveyor belt 130) may be imaged periodically for use in accounting for the variable and uncontrolled illumination, such as between piles of material resting on the conveyor belt. In some examples, the reference object is a surface on which a conveyance used to carry the material into the image location rests. For example, the reference object may form part of the surface of the haul truck route in a mining application. In some examples, the reference object is a surface of the conveyance that is used to carry the material (e.g., the overhang portion of the truck body). The reference object may be, e.g., a slab of a durable material forming part of the surface of the haul truck route. In some examples, the reference object 780 is a surface of packed soil forming part of the haul truck route.

It will be appreciated that the reflectance characteristics of the reference object 780 may be calculated, acquired from a database, or determined experimentally, and may periodically be updated. For example, the reflectance characteristics of a surface of packed soil may be determined by illuminating the surface of packed soil with a known illumination (e.g., artificial lights applied at night) and measuring the intensity of reflected light.

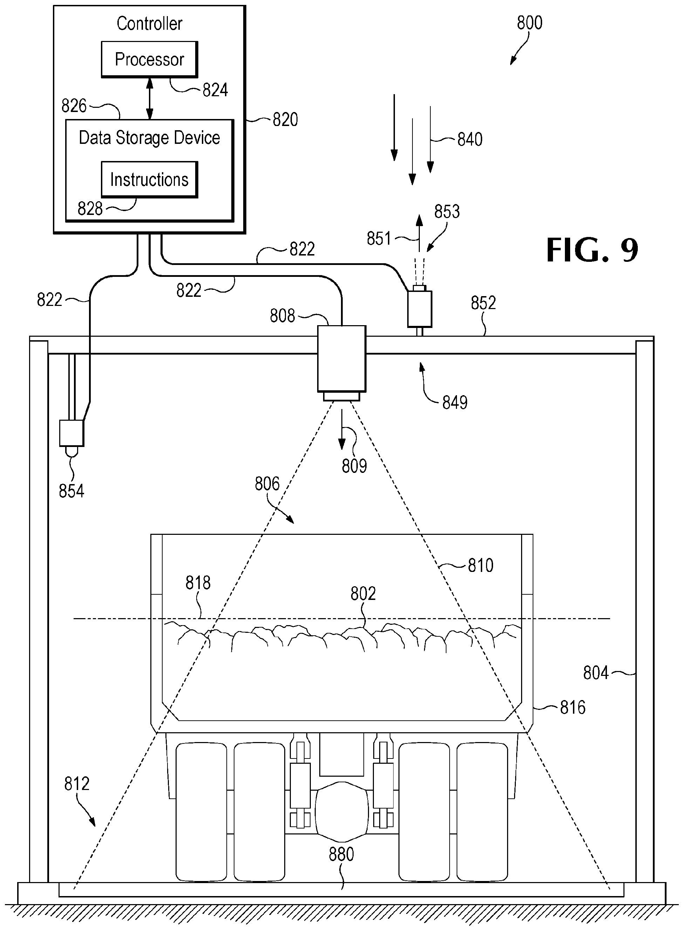

Referring to Figure 9, an imaging system 800 is illustrated that is substantially similar to the imaging system 100 of Figs. 1-2 with the noted exceptions, and like components are indicated by like reference numbers incremented by 700. As illustrated in Figure 9, the imaging system 800 may include a reference object and a reference device.

As illustrated in Figure 9, a spectral imager 808 is secured to a support structure 804 above a material transport path 812. The spectral imager 808 has a field of view 810 directed in first direction 809 (e.g., downward), such as towards a haul truck 816 traveling along the material transport path 812. The spectral imager 808 may be a push broom scanner with an imaging line 818 at an image location 806 arranged to image material 802 carried along the path 812. The system 800 may include artificial light source 854 to illuminate the image location 806. A reference object 880 is in the field of view 810 of the spectral imager 808.

The imaging system 800 further includes a reference device 849. The reference device 849 is directed in a second direction 851 different from the first direction 809. The reference device 849 is operable to sample the varying and uncontrolled illumination 840 to which the image location 806 is exposed for each of the plurality of wavelength bands of interest.

A controller 820 controls operations and/or receives information from the spectral imager 808 and/or the reference device 849 (e.g., communicatively coupled via a communications network 822). In some examples, the controller 820 includes at least one processor 824 and at least one data storage device 826. The at least one data storage device 826 stores a set of computer-executable instructions 828 for operating the at least one processor 824.

The set of instructions 828 include instructions to direct the spectral imager 808 to capture spectral imager data of an earthen material 802 at the image location 806. The spectral imager data of the earthen material includes the intensity of illumination reflected from the earthen material for each of the plurality of bands of interest. The set of instructions 828 include instructions to receive the spectral imager data associated with the earthen material from the spectral imager 808.

The set of instructions 828 include instructions to direct the spectral imager 808 to capture spectral imager data of the reference object 880. The spectral imager data of the reference object 880 includes the intensity of illumination reflected from the reference object 880 for each of the plurality of bands of interest. The set of instructions 828 include instructions to receive the spectral imager data associated with the reference object 880 from the spectral imager 808.

The set of instructions 828 include instructions to direct the capture of illumination data from a sample of the varying and uncontrolled illumination 840 sampled by the at least one reference device 849. The illumination data includes the intensity of the varying and uncontrolled illumination 840 for each of the plurality of bands of interest. The set of instructions 828 include instructions to receive the illumination data.

The set of instructions 828 include instructions to identify intensity variations among pixels associated with shadowing in the plurality of bands of interest captured in the spectral imager data associated with the reference object 880 from the spectral imager 808. The set of instructions 828 include instructions to apply the illumination data as the intensity of incident light at the image location 806 for each of the plurality of bands of interest.

The set of instructions 828 include instructions to determine the percentage of intensity of incident light at the pixel locations associated with shadowing in the plurality of bands of interest captured in the spectral imager data associated with the reference object 880 from the spectral imager 808. The set of instructions 828 include instructions to apply the spectral imager data of the material 802 and the intensity of incident light per pixel to determine a reflectance of the earthen material 802 for each of the plurality of bands of interest, wherein those pixels associated with shadowing in the plurality of bands of interest captured in the spectral imager data associated with the reference object 880 from the spectral imager 808 as adjusted by the percentage of intensity of incident light.

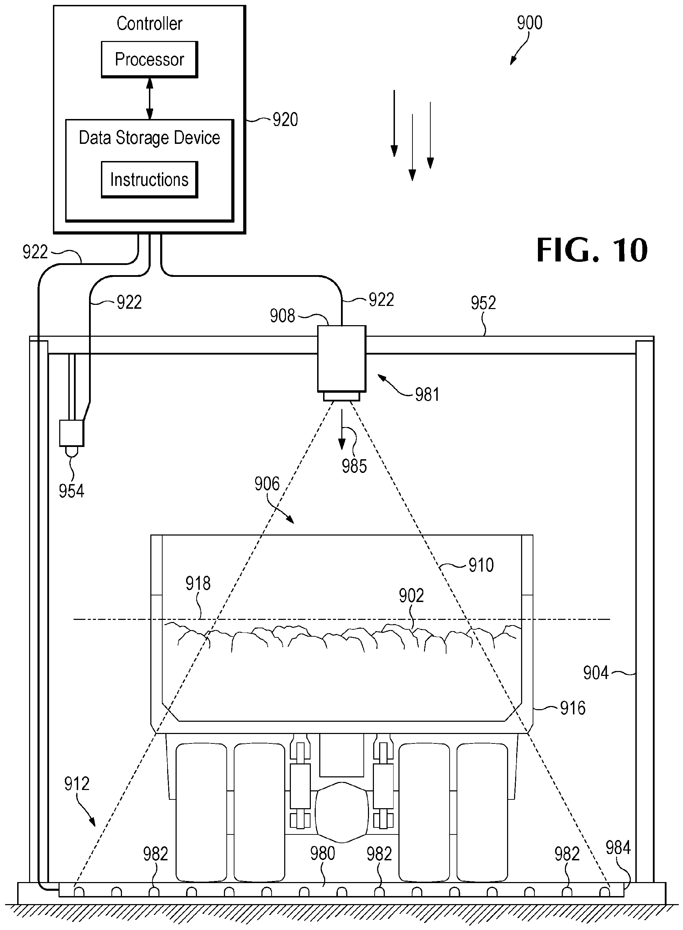

Referring to Figure 10, an imaging system 900 is illustrated that is substantially similar to the imaging system 100 of Figs. 1-2 with the noted exceptions, and like components are indicated by like reference numbers incremented by 800. As illustrated in Figure 10, a reference object may be an active reference object.

As exemplified in Figure 10, a spectral imager 908 is secured to a support structure 904 above a material transport path 912. The spectral imager 908 has a field of view 910. The spectral imager 908 may be a push broom scanner with an imaging line 918 at an image location 906 arranged to image material 902 carried along the path 912 (e.g., by haul truck 916). The system 900 may include artificial light source 954 to illuminate the image location 906.

The reference object 980 may be an active object. The reference object 980 may include one or more artificial illumination sources 982 (e.g., a plurality of artificial illumination sources 982) positioned within the field of view 910 of the spectral imager 908 and generating a known illumination (e.g., measured or calculated). As exemplified, a controller 920 may be communicatively coupled to the sources 982 (e.g., via network 922) to control operation of the sources 982 (e.g., according to a schedule or in response to an input such as a motion detection). In some examples, an active object may be used to determine if shadows are present, such as by comparing the measured light intensity coming from the plurality of light sources to the anticipated and known light intensity. In some examples, an active object may be used to determine if the reflectively of the object has changed, such as due to the presence of water or accumulated dust. Changing reflectivity of the reference object 980 may be detected using the known amount of illumination generated by the source(s) 982 compared to the amount received by the spectral imager 908. With the mix of known and unknown illumination, the unknown value can be determined by subtracting the known illumination amount from the total illumination received at the spectral camera, which may be done before an earthen material image capture was done to identify the ambient, uncontrolled illumination. The plurality of artificial illumination sources 982 may be arranged within a protective housing 984, as exemplified.

Referring to Figure 11, an imaging system 1000 is illustrated that is substantially similar to the imaging system 100 of Figs. 1-2 with the noted exceptions, and like components are indicated by like reference numbers incremented by 900. As illustrated in Figure 11, the spectral imager may be moveable between the first time and the second time.

As exemplified in Figure 11, a spectral imager 1008 is secured to a support structure 1004 above a material transport path 1012. The spectral imager 1008 has a field of view 1010. The spectral imager 1008 may be a push broom scanner with an imaging line 1018 at an image location 1006 arranged to image material 1002 carried along the path 1012 (e.g., by haul truck 1016). The system 1000 may include artificial light source 1054 to illuminate the image location 1006.

The spectral imager may be moveable between a first position 981 (Figure 10) and a second position 1083 (Figure 11). In the first position 981 the spectral imager is directed in a first direction 985 at the image location. In the first position 981 the spectral imager may be focused on the image location. In the second position 1083, the spectral imager 1008 is directed in a second direction 1087 that is different from the first direction 985 (Figure 10). The second direction 1087 may be generally opposite to the first direction 985. The second direction 1087 may be generally upwards, as exemplified in Figure 11. The first direction 985 may be generally downwards. Movement between the first and second positions may be rotational movement. The system 1000 may include an actuator 1089 coupled to the spectral imager 1008 to move the spectral imager 1008 between positions.

The spectral imager 1008 may be in the first position at the first time and in the second position 1083 at the second time. The spectral imager 1008 may be focused at the same distance from the spectral imager in the first and second positions or may be focused on difference distances. The second time may give the irradiance of the whole image surface (e.g., the image line) at a known distance from the image location at which the material is imaged. This known distance may be two times the distance between the spectral imager 1008 and the image location if the focus of the spectral imager 1008 is generally unchanged, since flipping the imager 1008 causes the imager 1008 to focus on a location that is the same distance away but in the opposite direction. It will be appreciated that data captured by the spectral imager 1008 at the second time in the second position may be used to determine an intensity of the varying and uncontrolled illumination at the second time.

In some examples in which spectral imager data of the reference from a second time is used (e.g., used to determine the intensity of incident light at the reference object at the second time or directly capturing the intensity of incident light at the sample location at the second time using the spectral imager in the second position), the set of instructions also includes instructions to apply the intensity of the varying and uncontrolled illumination at the second time to infer an intensity of incident light at the earthen material at the first time for use in calculating the reflectance of the material (which may also include taking into account controlled and/or non-variable light).

Applying the intensity of the varying and uncontrolled illumination at the second time to infer the intensity of incident light at the earthen material at the first time may be done in one of various ways. In some examples, the intensity of incident light at the earthen material at the first time is equated to the intensity as measured or calculated at the second time (e.g., corrected for any known artificial illumination differences). Alternatively, the intensity of incident light at the earthen material at the first time is equated to a modification of the intensity as measured or calculated at the second time, such as the intensity as measured or calculated at the second time as modified by a scalar value or an equation. Other configurations are possible.

In some examples, multiple reference images are used to infer the intensity of light at the image location at the first time. Applying the intensity of the varying and uncontrolled illumination at the second time to infer the intensity of incident light at the earthen material at the first time may involve combining the intensity of the varying and uncontrolled illumination at the second time with intensity of the varying and uncontrolled illumination at one or more further times, such as by using an average of intensities from a time before and from a time after the first time.

The set of instructions may include instructions to repeat one or more acquisition steps and/or application steps for different materials (e.g., different loads of mining material) and/or different images of the same material. In some examples, an inferred intensity of incident light at the image location is used for more than one set of spectral imager data taken by the spectral imager, such as for a series of sequential images taken by the spectral imager. The set of instructions may include instructions to acquire a new set of spectral imager data of the reference from the spectral imager and apply the new set of spectral imager data of the reference to infer the intensity of incident light at the image location according to a schedule or in response to an input, such as sensing a change in solar illumination.

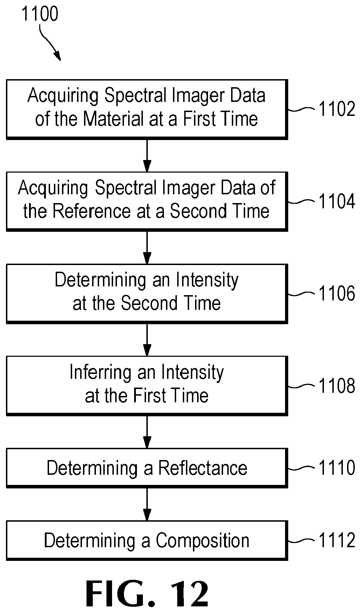

Referring to Figure 12, an imaging method 1100 using data acquired at a second time (e.g., from a reference object or before and/or after moving the spectral imager). The imaging method 1100 may be a computer implemented method. Method 1100 may be carried out by an imaging system including a reference object and a spectral imager that may be moveable to at least two different positions. Method 1100 may be carried out by a portion of an imaging system disclosed herein, such as by the controller.

The imaging method 1100 includes, at step 1102, acquiring, at a first time, spectral imager data of a material at an image location exposed to varying and uncontrolled illumination and/or artificial illumination. The spectral imager data of the material includes the intensity of illumination reflected from the material for each of a plurality of bands of interest. The material may be an earthen material, and the imaging method 1100 may be an earthen material imaging method.

At step 1104, method 1100 includes acquiring, at a second time, reference spectral imaging data of a reference. The reference spectral imaging data of the reference may be, e.g., of a reference object in a field of view. The spectral imaging data of the reference may be captured by the spectral imager used in step 1102 after the spectral imager has been moved to a new position. The reference spectral imaging data of the reference includes the ratio of illumination reflected from the reference objectfor the plurality of bands of interest, such as the ratio of illumination reflected from the reference object for each of the plurality of bands of interest. The second time is different from the first time. The first time may be subsequent or prior to the second time. The second time may be spaced apart from the first time or consecutive. In some examples, the method also includes simultaneously sensing the atmospheric lighting conditions and processing the captured hyperspectral image to account for lighting conditions. In other examples aerosol and/or atmospheric sensors may be used to estimate radiance. The information could also be retrieved online based on location of the sun at the mine site, weather, satellite atmospheric data, humidity data, temperature, and the like. In some cases, a dynamic offset correction method may be utilized if the reference object is not available, but the radiative transfer model is from the gathered atmospheric parameters, readings, data, or extrapolated data gathered from other sources. In some examples, the method may include a RGB camera to detect cloud cover.

At step 1106, method 1100 includes applying the spectral imaging data of the reference to determine an intensity of the varying and uncontrolled illumination at the second time. If artificial illumination is used, then the known intensity of the artificial illumination can be accounted for. For example, reflected artificial illumination would be a part of the data collected in step 1102. This known amount can be subtracted from the total amount of reflected illumination received at step 1102 to determine the remainder due to the variable and uncontrolled illumination. Step 1106 may include applying predetermined reflectance characteristics of the reference object to determine an intensity of incident light at the reference device at the second time.

At step 1108, method 1100 includes applying the intensity of the varying and uncontrolled illumination at the second time to infer an intensity of incident light at the earthen material at the first time. Method 1100 also includes, at step 1110, applying the inferred intensity of incident light at the earthen material at the first time and the spectral imager data of the material to determine a reflectance of the earthen material.

Method 1100 may also include, at step 1112, comparing the reflectance of the material to stored reflectance data for known materials to determine a composition of the material. Determining a composition may include identifying a particular element or elements found in the material or, also or alternatively, determining one or more material properties (e.g., illite crystallonity index, hardness, moisture content, and the like). Step 1112 may include comparing the reflectance of the material to a library of reflectance data for known materials or inputting the reflectance of the material to a machine learned model.

Method 1100 as a whole, or steps 1102, 1110, and 1112 thereof, may be repeated for different materials (e.g., different loads of mining material) and/or different images of a material. In some examples, the intensity of the varying and uncontrolled illumination at the second time is used to infer the intensity of incident light at the material at the first time and also at additional discrete times at which different materials are imaged and/or different images of a material are taken. In some examples, an intensity of incident light at the image location inferred at step 1108 is used for more than one set of spectral imager data acquired at step 1102, such as for a series of sequential images taken by the spectral imager. Steps 1104, 1106, and 1108 may be performed less frequently than steps 1102, 1110, and 1112. In some examples, steps 1104, 1106, and 1108 may be performed according to a schedule (e.g., a predetermined period of time or a predetermined number of instances of step 1102) or in response to an input, such as sensing a change in the varying and uncontrolled illumination (e.g., solar).

What has been described above is intended to be illustrative of the disclosure and non-limiting and it will be understood by persons skilled in the art that other variants and modifications may be made without departing from the scope of the disclosure as defined in the claims appended hereto. The scope of the claims should not be limited by the preferred examples and examples, but should be given the broadest interpretation consistent with the description as a whole. Features discussed above may be in different combinations than those discussed.

## Claims

1. An imaging system for earthen material, comprising:
a. a support structure adjacent an image location exposed to varying and uncontrolled illumination;
b. a spectral imager mounted to the support structure, the spectral imager directed in a first direction at the image location and operable to measure an intensity of illumination reflected from the earthen material at the image location for each of a plurality of wavelength bands of interest; and
c. at least one reference device directed in a static second direction different from the first direction, the at least one reference device operable to sample the varying and uncontrolled illumination to which the image location is exposed for one or more reference wavelength bands.

2. An imaging system for earthen material, comprising:
a. a support structure adjacent an image location exposed to varying and uncontrolled illumination;
b. a spectral imager mounted to the support structure, the spectral imager directed at and focused on the image location and operable to measure an intensity of illumination reflected from the image location for each of a plurality of bands of interest; and
c. a reference object in a field of view of the spectral imager beyond the image location.

3. An imaging system for earthen material, comprising:
a. a support structure adjacent an image location exposed to varying and uncontrolled illumination;
b. a spectral imager mounted to the support structure, the spectral imager directed in a first direction at the image location and operable to measure an intensity of illumination reflected from the earthen material at the image location for each of a plurality of wavelength bands of interest; and
c. first and second reference devices directed in a second direction different from the first direction, the second reference device located below the first reference device to directly monitor the image location area for a shadow of the support structure, the first and second reference devices operable to sample the varying and uncontrolled illumination to which the image location is exposed for each of one or more reference wavelength bands at two different locations.

4. An imaging system for earthen material, comprising:
a. a support structure adjacent an image location exposed to varying and uncontrolled illumination;
b. a spectral imager mounted to the support structure, the spectral imager directed in a first direction at the image location and operable to measure an intensity of illumination reflected from the earthen material at the image location for each of a plurality of wavelength bands of interest; and
c. at least one reference device directed in a second direction different from the first direction, the at least one reference device operable to sample the varying and uncontrolled illumination to which the image location is exposed for one or more reference wavelength bands; and
d. a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to:
i. direct the spectral imager to capture spectral imager data of an earthen material at the image location, the spectral imager data including the intensity of illumination reflected from the earthen material for each of the plurality of bands of interest;
ii. receive the spectral imager data from the spectral imager;
iii. direct the capture of illumination data from a sample of the varying and uncontrolled illumination sampled by the at least one static reference device, the illumination data including the intensity of the varying and uncontrolled illumination for the one or more reference wavelength bands;
iv. receive the illumination data;
v. apply the illumination data to determine an intensity of incident light at the image location for each of the plurality of bands of interest; and
vi. apply the spectral imager data and the intensity of incident light to determine a reflectance of the earthen material for each of the plurality of bands of interest.

5. An imaging system for earthen material, comprising:
a. a support structure adjacent an image location exposed to varying and uncontrolled illumination;
b. a spectral imager mounted to the support structure, the spectral imager directed at the image location and operable to measure an intensity of illumination reflected from the image location for each of a plurality of bands of interest;
c. a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to:
i. direct the spectral imager to capture spectral imager data of an earthen material at the image location at a first time, the spectral imager data including the intensity of illumination reflected from the earthen material for each of the plurality of bands of interest;
ii. receive the spectral imager data from the spectral imager;
iii. direct the spectral imager to capture spectral imager data of a reference at a second time, the second time being different from the first time and the spectral imager data of the reference including intensity for one or more reference wavelength bands;
iv. receive the spectral imager data of the reference from the spectral imager;
v. apply the spectral imager data of the reference to determine an intensity of the varying and uncontrolled illumination at the second time for the one or more reference wavelength bands;
vi. apply the intensity of the varying and uncontrolled illumination at the second time to infer an intensity of incident light at the earthen material at the first time for each of the plurality of bands of interest; and
vii. apply the intensity of incident light at the earthen material at the first time for each of the plurality of bands of interest and the spectral imager data to determine a reflectance of the earthen material.

6. An imaging method for earthen material, comprising:
a. acquiring, at a first time, spectral imager data of an earthen material at an image location exposed to varying and uncontrolled illumination, the spectral imager data captured by a spectral imager directed at and focused on the image location and including the intensity of illumination reflected from the earthen material for each of a plurality of bands of interest;
b. acquiring, at a second time, spectral imager data of a reference from a field of view of the spectral imager, the second time being different from the first time and the spectral imager data of the reference including the intensity for one or more reference wavelength bands;
c. applying the spectral imager data of the reference to determine an intensity of the varying and uncontrolled illumination at the second time for the one or more reference wavelength bands;
d. applying the intensity of the varying and uncontrolled illumination at the second time to infer an intensity of incident light at the earthen material at the first time for each of the plurality of bands of interest; and
e. applying the intensity of incident light at the earthen material at the first time for each of the plurality of bands of interest and the spectral imager data to determine a reflectance of the earthen material.

7. An imaging system for earthen material, comprising:
a. a support structure adjacent an image location exposed to varying and uncontrolled illumination;
b. a spectral imager mounted to the support structure, the spectral imager directed in a first direction at the image location and operable to measure an intensity of illumination reflected from the earthen material at the image location for each of a plurality of wavelength bands of interest;
c. first and second reference devices directed in a second direction different from the first direction, the second reference device located below the first reference device to directly monitor the image location area for a shadow of the support structure, the first and second reference devices operable to sample the varying and uncontrolled illumination to which the image location is exposed for each of one or more reference wavelength bands at two different locations, and
d. a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to:
i. direct the spectral imager to capture spectral imager data of an earthen material at the image location, the spectral imager data including the intensity of illumination reflected from the earthen material for each of the plurality of bands of interest;
ii. receive the spectral imager data from the spectral imager;
iii. direct the first reference device to capture first illumination data from a sample of the varying and uncontrolled illumination sampled from the first reference device, the first illumination data including the intensity of the varying and uncontrolled illumination for the one or more reference wavelength bands at a first location;
iv. direct the second reference device to capture second illumination data from a sample of the varying and uncontrolled illumination sampled by the second reference device, the second illumination data including the intensity of the varying and uncontrolled illumination for the one or more reference wavelength bands at a second location;
v. receive the first and second illumination data;
vi. apply the first and second illumination data to determine a difference in an intensity of incident light at the image location for the one or more reference wavelength bands;
vii. if the first and second illumination data are different, then apply the spectral imager data and the intensity of incident light affected by shadow cover to determine a reflectance of the earthen material for each of the plurality of bands of interest, otherwise apply the spectral imager data and the intensity of incident light associated with the first and second illumination data to determine a reflectance of the earthen material for each of the plurality of bands of interest.

8. An imaging system for earthen material, comprising:
a. a support structure adjacent an image location exposed to varying and uncontrolled illumination;
b. a spectral imager mounted to the support structure, the spectral imager directed at and focused on the image location and operable to measure an intensity of illumination reflected from the image location for each of a plurality of bands of interest;
c. a reference object in a field of view of the spectral imager beyond the image location,
d. at least one reference device directed in a second direction different from the first direction, the at least one reference device operable to sample the varying and uncontrolled illumination to which the image location is exposed for each of one or more reference wavelength bands, and
e. a controller communicatively coupled to the spectral imager to control operations of the spectral imager and receive data from the spectral imager, the controller including at least one processor and at least one data storage device communicatively coupled to the at least one processor and having stored thereon computer-executable instructions for operating the at least one processor to:
i. direct the spectral imager to capture spectral imager data of an earthen material at the image location, the spectral imager data including the intensity of illumination reflected from the earthen material for each of the plurality of bands of interest;
ii. receive the spectral imager data associated with the earthen material from the spectral imager;
iii. direct the spectral imager to capture spectral imager data of the reference object, the spectral imager data including the intensity of illumination reflected from the reference object for the one or more reference wavelength bands;
iv. receive the spectral imager data associated with the reference object from the spectral imager;
v. direct the capture of illumination data from a sample of the varying and uncontrolled illumination sampled by the at least one reference device, the illumination data including the intensity of the varying and uncontrolled illumination for the one or more reference wavelength bands;
vi. receive the illumination data;
vii. identifying intensity variations among pixels associated with shadowing in the one or more reference wavelength bands captured in the spectral imager data associated with the reference object from the spectral imager;
viii. apply the illumination data as the intensity of incident light at the image location for each of the plurality of bands of interest;
ix. determine the percentage of intensity of incident light at the pixel locations associated with shadowing in the plurality of bands of interest captured in the spectral imager data associated with the reference object from the spectral imager; and
x. apply the spectral imager data and the intensity of incident light per pixel to determine a reflectance of the earthen material for each of the plurality of bands of interest, wherein those pixels associated with shadowing in the plurality of bands of interest captured in the spectral imager data associated with the reference object from the spectral imager as adjusted by the percentage of intensity of incident light.

9. The imaging system of any one of claims 1-8, wherein the one or more reference wavelength bands include the plurality of wavelength bands of interest.

10. The imaging system of any one of claims 1-9, wherein the second direction is generally opposite to the first direction.

11. The imaging system of any one of claims 1-10, wherein each of the at least one reference device is arranged to sample the varying and uncontrolled illumination at a location that is spaced from the image location.

12. The imaging system of any one of claims 1-11, wherein the support structure extends over the image location, the spectral imager is directed downward towards the image location, and the at least one reference device is directed upwards away from the image location.

13. The imaging system of claim 12, wherein the image location is a haul truck route for a mine site, and the support structure extends over the haul truck route and supports the spectral imager above the haul truck route.

14. The imaging system of claim 12, wherein the image location is a conveyer belt for an earthen material processing plant, and the support structure extends over the conveyer belt and supports the spectral imager above the conveyer belt.

15. The imaging system of any one of claims 1-14, wherein the at least one reference device is a plurality of reference devices to sample the varying and uncontrolled illumination from a plurality of spaced-apart sample locations.

16. The imaging system of any one of claims 1-15, wherein the spectral imager is a hyperspectral camera operable to measure hyperspectral spectral imager data of the material.

17. The imaging system of claim 16, wherein the hyperspectral camera is a line scanning hyperspectral camera and the at least one reference device is a plurality of reference devices to sample the varying and uncontrolled illumination from a plurality of spaced-apart sample locations, the sample locations arranged in a linear arrangement.

18. The imaging system of any one of claims 1-17, wherein a material movement plane is a plane extending through the image location and generally perpendicular to a straight line from the spectral imager to a center of the image location, and each of the at least one reference device is across the material movement plane from the spectral imager.

19. The imaging system of any one of claims 1-18, wherein each of the at least one reference device is located below the spectral imager.

20. The imaging system of any one of claims 1-19, wherein each of the at least one reference device is mounted to the support structure and directed away from the image location.

21. The imaging system of claim 20, wherein the spectral imager is mounted to a portion of the support structure that is above the image location, and one or more device of the at least one reference device is mounted above the portion of the support structure to which the spectral imager is mounted.

22. The imaging system of claim 20, wherein the spectral imager is mounted to a portion of the support structure that is above the image location, and one or more device of the at least one reference device is mounted between the image location and the portion of the support structure to which the spectral imager is mounted.

23. The imaging system of any one of claims 1-22, further comprising at least one artificial light source mounted to the support structure and arranged to provide controlled illumination with a known intensity to the image location, and wherein determining an intensity of incident light at the image location includes accounting for the controlled illumination with a known intensity and the varying and uncontrolled illumination.

24. The imaging system of claim 23, wherein the controller is operably coupled to the at least one artificial light source to control when the at least one artificial light source is on and when the at least one artificial light source is off.

25. The imaging system of claim 24, wherein the controller is operable to switch the at least one artificial light source between off and on according to a schedule stored on the at least one data storage device.

26. The imaging system of claim 24, wherein the controller is operable to switch the at least one artificial light source on in response to detection of a shadow at the image location.

27. The imaging system of claim 26, wherein the at least one reference device is not exposed to the controlled artificial light source.

28. The imaging system of claim 27, wherein the at least one data storage device stores emission characteristics of the at least one artificial light source and spacing information indicative of spacing between the at least one artificial light source and the spectral imager, and wherein accounting for the controlled illumination includes accessing and processing the emission characteristics and spacing information.

29. The imaging system of any one of claims 1-28, wherein each of the at least one reference device is positioned in-line with the image location and a portion of the support structure to which the spectral imager is mounted, whereby a shadow cast by the portion of the support structure on the image location is experienced by each of the at least one reference device.

30. The imaging system of any one of claims 1-29, wherein each of the at least one reference device includes a reference sensor operable to measure an intensity of the varying and uncontrolled illumination to which the image location is exposed for each of the plurality of wavelength bands of interest.

31. The imaging system of any one of claims 4-29, wherein each of the at least one reference device includes a reference sensor operable to measure an intensity of the varying and uncontrolled illumination to which the image location is exposed for each of the plurality of wavelength bands of interest, and wherein the controller is communicatively coupled to the at least one reference device to control operations thereof and receive data therefrom, and the illumination data is captured by the at least one reference sensor.

32. The imaging system of claim 31, wherein the reference sensor is a spectrometer.

33. The imaging system of any one of claims 1-32, further comprising a controller communicatively coupled to the spectral imager and the at least one reference device to direct operations of the spectral imager and the at least one reference device and to receive data from the spectral imager and the at least one reference device.

34. The imaging system of any one of claims 1-33, wherein each of the at least one reference device is an optical system that forms an optical path to redirect a sample of the varying and uncontrolled illumination to which the image location is exposed, the optical path including a path inlet end directed in the second direction and a path outlet end in the field of view of the spectral imager and directed towards the spectral imager.

35. The imaging system of claim 34, wherein each reference device includes a fiber optic cable having a cable inlet end forming the path inlet end and a cable outlet end forming the path outlet end.

36. The imaging system of any one of claims 1-35, wherein the reference device is a component of the spectral imager.

37. The imaging system of any one of claims 1-36, further comprising at least one processor in communication with the spectral imager, the at least one processor being operably configured to select at least one spectral image from the successively captured images in response to a likelihood of a vehicle and load carrying container being within the field of view of the spectral imager.

38. The imaging system of any one of claims 1-37, wherein the image location includes a pathway for at least a portion of a load container of a haul truck, a railcar, a barge, a trolley, a LHD vehicle, and/or a mining skip.

39. The imaging system of claim 1-38, further comprising at least one of an atmospheric sensor, irradiance sensor, photometer, temperature sensors, to determine incident radiance at the location and correct the intensity of illumination reflected from the reference object when the reference object is unavailable due to environmental conditions.

40. The imaging system of claim 4-39, wherein the spectral imager is operable to move between a first position and a second position, the first position directed in a first direction at and focused on the image location, and the second position directed in a second direction different from the first direction, and the controller include instructions to move the spectral imager between the first position and the second position between the first time and the second time.

41. The imaging system of any one of claims 2 and 8, wherein the spectral imager data and the illumination data are captured generally simultaneously.

42. The imaging system of any one of claims 2 and 8, wherein the wherein the spectral imager data and the illumination data are captured generally sequentially.

43. The imaging system of any one of claims 2 and 8, wherein the reference object is a surface of packed soil forming part of the haul truck route.

44. The imaging system of any one of claims 2 and 8, wherein the reference object is a surface of a slab of concrete or metal.

45. The imaging system of any one of claims 1 and 3-7, further comprising:
a. a reference object in the field of view of the spectral imager beyond the image location,
wherein the data storage device stores predetermined reflectance characteristics of the reference object, and
wherein the spectral imager data of the reference is of the reference object and the intensity for the one or more reference wavelength bands at the second time is the intensity of illumination reflected from the reference object, and applying the spectral imager data of the reference to determine an intensity of the varying and uncontrolled illumination at the second time includes applying the reflectance characteristics of the reference object.

46. The imaging system of claim 45, wherein the reference object is in the field of view of the spectral imager beyond the image location on which the spectral imager is focused at the first time and at the second time.

47. The imaging system of claim 45, wherein the reference object includes a plurality of artificial illumination sources positioned within the field of view and generating a known illumination, and applying the spectral imager data of the reference and the reflectance characteristics of the reference object to determine the intensity of incident light at the reference object at the second time includes subtracting the known illumination from the spectral imager data of the reference to determine reference reflectance data representing the variable and uncontrolled illumination reflected by the reference object.
